(19) Europäisches Patentamt
European Patent Office
Office européen des brevets

(11) EP 2 049 525 B1

(12) # FASCICULE DE BREVET EUROPEEN

(45) Date de publication et mention de la délivrance du brevet:
**30.12.2009 Bulletin 2009/53**

(21) Numéro de dépôt: **07823371.5**

(22) Date de dépôt: **30.07.2007**

(51) Int Cl.:
***C07D 401/14*** *(2006.01)* ***C07D 401/12*** *(2006.01)*
***A61K 31/4439*** *(2006.01)* ***A61P 29/00*** *(2006.01)*
***A61P 25/24*** *(2006.01)* ***A61P 25/04*** *(2006.01)*
***A61P 17/00*** *(2006.01)* ***A61P 15/00*** *(2006.01)*
***A61P 13/00*** *(2006.01)* ***A61P 11/00*** *(2006.01)*
***A61P 3/10*** *(2006.01)* ***C07D 213/74*** *(2006.01)*
***C07D 401/04*** *(2006.01)* ***C07D 403/12*** *(2006.01)*

(86) Numéro de dépôt international:
**PCT/FR2007/001316**

(87) Numéro de publication internationale:
**WO 2008/015335 (07.02.2008 Gazette 2008/06)**

(54) **DERIVES DE N-(AMINO-HETEROARYL)-1H-INDOLE-2-CARBOXAMIDES, LEUR PREPARATION ET LEUR APPLICATION EN THERAPEUTIQUE**

N-(AMINOHETEROARYL)-1H-INDOL-2-CARBOXAMID-DERIVATE SOWIE DEREN HERSTELLUNG UND THERAPEUTISCHE ANWENDUNG

N-(AMINOHETEROARYL)-1H-INDOLE-2-CARBOXAMIDE DERIVATIVES, AND PREPARATION AND THERAPEUTIC APPLICATION THEREOF

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **31.07.2006 FR 0606988**

(43) Date de publication de la demande:
**22.04.2009 Bulletin 2009/17**

(73) Titulaire: **Sanofi-Aventis**
**75013 Paris (FR)**

(72) Inventeurs:
- **DUBOIS, Laurent**
**75013 Paris (FR)**
- **EVANNO, Yannick**
**75013 Paris (FR)**
- **MALANDA, André**
**75013 Paris (FR)**
- **MACHNIK, David**
**75013 Paris (FR)**
- **GILLE, Catherine**
**75013 Paris (FR)**

(74) Mandataire: **Morel-Pécheux, Muriel et al**
**sanofi-aventis**
**Département Brevets**
**174 avenue de France**
**75013 Paris (FR)**

(56) Documents cités:
**WO-A-2006/072736 WO-A-2007/060140**

- **BRANDS ET AL: "Novel, selective indole-based ECE inhibitors: Lead optimization via solid-phase and classical synthesis" BIOORGANIC & MEDICINAL CHEMISTRY LETTERS, OXFORD, GB, vol. 15, no. 19, 1 octobre 2005 (2005-10-01), pages 4201-4205, XP005038874 ISSN: 0960-894X**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet des composés dérivés de *N*-(amino-hétéroaryl)-1H-indole-2-carboxamides, qui présentent une activité antagoniste *in vitro* et *in vivo* pour les récepteurs de type TRPV1 (ou VR1).

WO 2006/072736 décrit des dérivés de N-(hétéroaryl)-1*H*-indole-2-carboxamides utiles comme ligands du recepteur vanilloide TRPV1.

Un premier objet de l'invention concerne les composés répondant à la formule générale (I) ci-après.
Un autre objet de l'invention concerne des procédés de préparation des composés de formule générale (I).
Un autre objet de l'invention concerne l'utilisation des composés de formule générale (I) notamment dans des médicaments ou dans des compositions pharmaceutiques.

Les composés de l'invention répondent à la formule générale (I) :

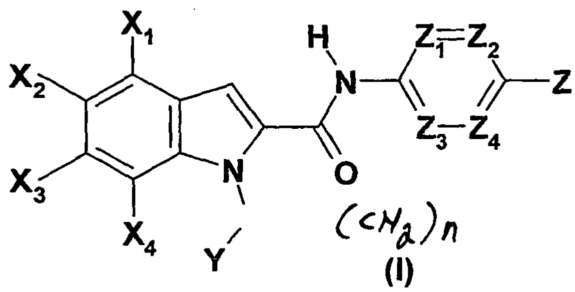

(I)

dans laquelle

| | |
|---|---|
| $X_1$ | représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, cyano, $C(O)NR_1R_2$, nitro, $C_1$-$C_6$-thioalkyle, -S(O)-$C_1$-$C_6$-alkyle, -$S(O)_2$-$C_1$-$C_6$-alkyle, $SO_2NR_1R_2$, aryle-$C_1$-$C_6$-alkylène, aryle ou hétéroaryle, l'aryle et l'hétéroaryle étant éventuellement substitués par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ; |
| $X_2$ | représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cydoalkyle-$C_1$-$C_6$-alkylène-O-, $C_1$-$C_6$-fluoroalcoxyle, cyano, $C(O)NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, aryle-$C_1$-$C_6$-alkylène, aryle ou hétéroaryle, l'aryle et l'hétéroaryle étant éventuellement substitués par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ; |
| $X_3$ et $X_4$ | représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$, aryle-$C_1$-$C_6$-alkylène, aryle ou hétéroaryle, l'aryle et l'hétéroaryle étant éventuellement substitués par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ; |
| $Z_1$, $Z_2$, $Z_3$, $Z_4$ | représentent, indépendamment l'un de l'autre, un atome d'azote ou un groupe $C(R_6)$, l'un au moins correspondant à un atome d'azote et l'un au moins correspondant à un groupe $C(R_6)$ ; l'atome d'azote ou un des atomes d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ; |
| n | est égal à 0, 1, 2 ou 3 ; |
| Y | représente un aryle ou un hétéroaryle éventuellement substitué par un ou plusieurs groupes choisis parmi un atome d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, hydroxyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C(O)NR_1R_2$, nitro, $NR_1R_2$, $C_1$-$C_6$-thioalkyle, thiol, -S(O)-$C_1$-$C_6$-alkyle, -$S(O)_2$-$C_1$-$C_6$-alkyle, $SO_2NR_1R_2$, $NR_3COR_4$, $NR_3SO_2R_5$, aryle-$C_1$-$C_6$-alkylène ou aryle, l'aryle et l'aryle-$C_1$-$C_6$-alkylène étant éventuellement substitué par un ou plusieurs substitutants choisi parmi un |

halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

Z représente une amine cyclique reliée par l'atome d'azote, de formule :

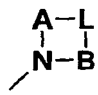

dans laquelle

A représente un groupe $C_1$-$C_7$-alkylène éventuellement substitué par un ou deux groupes $R_8$ ;
B représente un groupe $C_1$-$C_7$-alkylène éventuellement substitué par un ou deux groupes $R_9$ ;
L représente une liaison, un atome de soufre, d'oxygène ou d'azote ;
l'atome d'azote étant éventuellement substitué par un groupe $R_{10}$ ou $R_{11}$. les atomes de carbone de l'amine cyclique Z étant éventuellement substitués par un ou plusieurs groupes $R_{12}$ identiques ou différents l'un de l'autre ;

$R_1$ et $R_2$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle ; ou $R_1$ et $R_2$ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe $C_1$-$C_6$-alkyle, $C_3$-$C_7$-cycloalkyle, $C_3$-$C_7$-cydoalkyle-$C_1$-$C_3$-alkylène, aryle-$C_1$-$C_6$-alkylène, aryle ou hétéroaryle ;

$R_3$ et $R_4$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle ;

$R_5$ représente un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle ;

$R_6$ représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, $C_1$-$C_6$-thioalkyle, -S(O)-$C_1$-$C_6$-alkyle, -S(O)$_2$-$C_1$-$C_6$-alkyle, aryle, aryl-$C_1$-$C_6$-alkylène, hétéroaryle, hydroxyle, thiol, oxo ou thio ;

$R_7$ représente un atome d'hydrogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, aryle, aryl-$C_1$-$C_6$-alkylène ou hétéroaryle ;

$R_8$, $R_9$ et $R_{10}$ sont définis tels que :

deux groupes $R_8$ peuvent former ensemble une liaison ou un groupe $C_1$-$C_6$-alkylène ;
deux groupes $R_9$ peuvent former ensemble une liaison ou un groupe $C_1$-$C_6$-alkylène ;
$R_8$ et $R_9$ peuvent former ensemble une liaison ou un groupe $C_1$-$C_6$-alkylène ;
$R_8$ et $R_{10}$ peuvent former ensemble une liaison ou un groupe $C_1$-$C_6$-alkylène ;
$R_9$ et $R_{10}$ peuvent former ensemble une liaison ou un groupe $C_1$-$C_6$-alkylène ;

$R_{11}$ représente un atome d'hydrogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, hydroxyle, $COOR_5$, C(O)$NR_1R_2$, aryle-$C_1$-$C_6$-alkylène, aryle ou hétéroaryle, l'aryle et l'hétéroaryle étant éventuellement substitués par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;

$R_{12}$ représente un atome de fluor, un groupe $C_1$-$C_6$-alkyle éventuellement substitué par un groupe $R_{13}$, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-cycloalk-1,1-diyle, $C_3$-$C_7$-hétérocycloalk-1,1-diyle éventuellement substitué sur un atome d'azote par un groupe $R_{11}$, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, C(O)$NR_1R_2$, $NR_1R_2$, $NR_3COR_4$, OC(O)$NR_1R_2$, $NR_3COOR_5$, $NR_3CONR_1R_2$, hydroxyle, thiol, oxo, thio, aryle-$C_1$-$C_6$-alkylène, aryle, l'aryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;

$R_{13}$ représente un $C_1$-$C_6$-alcoxyle, $C_3$-$C_7$-cycloalcoxyle, $C_3$-$C_7$-cycloalkyle-$C_1$-$C_6$-alkylène-O-, C(O)

$NR_1R_2$, $NR_1R_2$, $NR_3COR_4$, $OC(O)NR_1R_2$, $NR_3COOR_5$, hydroxyle.

Dans les composés de formule générale (I), le ou les atomes d'azote peuvent être sous forme oxydée (N-oxyde). Dans les composés de formule générale (I), le ou les atomes de soufre peuvent être sous forme oxydée (S(O) ou $S(O)_2$).

A titre d'exemples non limitatifs d'amines Z, on peut citer l'aziridine, l'azétidine, la pyrrolidine, la pipéridine, l'azépine, la morpholine, la thiomorpholine, la pipérazine, l'homopipérazine, les azabicyclo[3.3.0]octanes, les octahydrofuropyrroles, les octahydropyrrolopyrroles, l'octahydroindole, l'octahydroisoindole, les octahydropyrrolopyridines, la décahydroquinoléine, la décahydroisoquinoléine, les décahydronaphthyridines, l'octahydropyridopyrazine, les azabicylo [3.1.0]hexanes, les azabicylo[3.2.0]heptanes, les azabicylo[3.1.1]heptanes, les diazabicylo[2.2.1]heptanes, les azabicylo[3.2.1]octanes, les diazabicylo[3.2.1]octanes, les azabicylo[3.3.1]nonanes.

Parmi les composés de formule générale (I) objets de l'invention, un premier sous-groupe de composés est constitué par les composés pour lesquels $X_1$, $X_2$, $X_3$ et $X_4$ sont choisis, indépendamment l'un de l'autre, parmi un atome d'hydrogène ou d'halogène ou un groupe $C_1$-$C_6$-alkyle, $C_1$-$C_6$-fluoroalkyle, $C_1$-$C_6$-thioalkyle, -$S(O)_2$-$C_1$-$C_6$-alkyle.

Parmi les composés de formule générale (I) objets de l'invention, un second sous-groupe de composés est constitué par les composés pour lesquels $X_1$, $X_2$, $X_3$ et $X_4$ sont choisis, indépendamment l'un de l'autre, parmi un atome d'hydrogène ou d'halogène ou un groupe $C_1$-$C_6$-alkyle ou $C_1$-$C_6$-fluoroalkyle.

Parmi les composés de formule générale (I) objets de l'invention, un troisième sous-groupe de composés est constitué par les composés pour lesquels $X_1$, $X_2$, $X_3$ et $X_4$ sont choisis, indépendamment l'un de l'autre, parmi un atome d'hydrogène, de fluor ou de chlore ou un groupe méthyle, éthyle, isopropyle, tertiobutyle, trifluorométhyle, thiométhyle, -$S(O)_2CH_3$.

Parmi les composés de formule générale (I) objets de l'invention, un quatrième sous-groupe de composés est constitué par les composés pour lesquels

$X_1$ représente un atome d'hydrogène ou d'halogène ou un groupe $C_1$-$C_6$-alkyle ;

$X_2$ représente un atome d'hydrogène ou d'halogène ou un groupe $C_1$-$C_6$-alkyle, $C_1$-$C_6$-fluoroalkyle, -$S(O)_2$-$C_1$-$C_6$-alkyle ;

$X_3$ et $X_4$ représentent, indépendamment l'un de l'autre, un atome d'hydrogène ou d'halogène ou un groupe $C_1$-$C_6$-alkyle, $C_1$-$C_6$-fluoroalkyle, $C_1$-$C_6$-alcoxyle, $NR_1R_2$, $C_1$-$C_6$-thioalkyle ;

$R_1$ et $R_2$ représentent, indépendamment l'un de l'autre, un groupe $C_1$-$C_6$-alkyle.

Parmi les composés de formule générale (I) objets de l'invention, un cinquième sous-groupe de composés est constitué par les composés pour lesquels

$X_1$ représente un atome d'hydrogène, de fluor ou de chlore ou un groupe méthyle ;

$X_2$ représente un atome d'hydrogène, de fluor ou de chlore ou un groupe méthyle, isopropyle, tertiobutyle, trifluorométhyle, -$S(O)_2CH_3$ ;

$X_3$ et $X_4$ représentent, indépendamment l'un de l'autre, un atome d'hydrogène, de fluor ou de chlore ou un groupe méthyle, éthyle, isopropyle, tertiobutyle, trifluorométhyle, -O-isopropyle, $NR_1R_2$, thiométhyle ;

$R_1$ et $R_2$ représentent, indépendamment l'un de l'autre, un groupe méthyle.

Parmi les composés de formule générale (I) objets de l'invention, un sixième sous-groupe de composés est constitué par les composés pour lesquels $Z_1$, $Z_2$, $Z_3$, $Z_4$ représentent, indépendamment l'un de l'autre, un atome d'azote ou un groupe $C(R_6)$, deux d'entre eux au moins correspondant à un groupe $C(R_6)$ ; l'atome d'azote ou un des atomes d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ; $R_6$ et $R_7$ étant tels que définis dans la formule générale (I).

Parmi les composés de formule générale (I) objets de l'invention, un septième sous-groupe de composés est constitué par les composés pour lesquels $Z_1$ et $Z_3$ représentent un groupe $C(R_6)$ et $Z_2$ et $Z_4$ représentent un atome d'azote ; $R_6$ étant tel que défini dans la formule générale (I).

Parmi les composés de formule générale (I) objets de l'invention, un huitième sous-groupe de composés est constitué par les composés pour lesquels $Z_1$ et $Z_3$ représentent un groupe $C(R_6)$ et $Z_2$ et $Z_4$ représentent un atome d'azote ; $R_6$ correspondant à un atome d'hydrogène.

Parmi les composés de formule générale (I) objets de l'invention, un neuvième sous-groupe de composés est constitué par les composés pour lesquels $Z_1$, $Z_2$, $Z_3$, $Z_4$ représentent, indépendamment l'un de l'autre, un atome d'azote ou un groupe $C(R_6)$, l'un correspondant à un atome d'azote et les autres correspondant à un groupe $C(R_6)$ ; l'atome d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ; $R_6$ et $R_7$ étant tels que définis dans la formule générale (I).

Parmi les composés de formule générale (I) objets de l'invention, un dixième sous-groupe de composés est constitué par les composés pour lesquels $Z_4$ représente un atome d'azote et $Z_1$, $Z_2$ et $Z_3$ représentent, indépendamment l'un de l'autre, un groupe $C(R_6)$ ; l'atome d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ; $R_6$ et $R_7$ étant tels que définis dans la formule générale (I).

Parmi les composés de formule générale (I) objets de l'invention, un onzième sous-groupe de composés est constitué par les composés pour lesquels $Z_4$ représente un atome d'azote et $Z_1$, $Z_2$ et $Z_3$ représentent, indépendamment l'un de l'autre, un groupe $C(R_6)$ ; $R_6$ représente un atome d'hydrogène ou un groupe $C_1$-$C_6$-alkyle, $C_1$-$C_6$-fluoroalkyle ou $C_1$-$C_6$-alcoxyle.

Parmi les composés de formule générale (1) objets de l'invention, un douzième sous-groupe de composés est constitué par les composés pour lesquels $Z_4$ représente un atome d'azote et $Z_1$, $Z_2$ et $Z_3$ représentent, indépendamment l'un de l'autre, un groupe $C(R_6)$ ; $R_6$ représente un atome d'hydrogène ou un groupe méthyle, trifluorométhyle ou méthoxy.

Parmi les composés de formule générale (I) objets de l'invention, un treizième sous-groupe de composés est constitué par les composés pour lesquels n est égal à 1.

Parmi les composés de formule générale (I) objets de l'invention, un quatorzième sous-groupe de composés est constitué par les composés pour lesquels Y représente un aryle ou un hétéroaryle éventuellement substitué par un ou plusieurs groupes choisi parmi un atome d'halogène ou un groupe $C_1$-$C_6$-alkyle ou $NR_1R_2$ ;
$R_1$ et $R_2$ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle.

Parmi les composés de formule générale (I) objets de l'invention, un quinzième sous-groupe de composés est constitué par les composés pour lesquels Y représente un phényle ou un pyridinyle, le phényle étant éventuellement substitué par un groupe choisi parmi un atome de fluor ou un groupe méthyle ou $NR_1R_2$ ;
$R_1$ et $R_2$ forment ensemble, avec l'atome d'azote qui les porte, un groupe pyrrolidinyle.

Parmi les composés de formule générale (I) objets de l'invention, un seizième sous-groupe de composés est constitué par les composés pour lesquels
Z représente une amine cyclique reliée par l'atome d'azote, de formule :

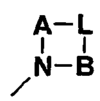

dans laquelle

- A représente un groupe $C_1$-$C_4$-alkylène éventuellement substitué par un ou deux groupes $R_8$ ;
- B représente un groupe $C_1$-$C_4$-alkylène éventuellement substitué par un ou deux groupes $R_9$ ;
- L représente une liaison ou un atome d'oxygène ;

l'atome d'azote de l'amine cyclique Z pouvant être sous forme N-oxyde ;
les atomes de carbone de l'amine cyclique Z étant éventuellement substitués par un groupe $R_{12}$ ;
$R_8$ et $R_9$ sont définis tels que :

- deux groupes $R_8$ peuvent former ensemble une liaison, ou
- deux groupes $R_9$ peuvent former ensemble une liaison ; ou
- $R_8$ et $R_9$ peuvent former ensemble une liaison ;

$R_{12}$ représente un groupe $NR_1R_2$, $NR_3COOR_5$, hydroxyle ;
$R_1$ et $R_2$, représentent, indépendamment l'un de l'autre, un atome d'hydrogène ;
$R_3$ représente un atome d'hydrogène ;
$R_5$ représente un groupe $C_1$-$C_6$-alkyle.

Parmi les composés de formule générale (I) objets de l'invention, un dix-septième sous-groupe de composés est constitué par les composés pour lesquels
Z représente une amine cyclique choisie parmi l'azétidine, la pyrrolidine, la pipéridine, la morpholine, l'azabicyclo[3.1.0]hexane et l'azabicyclo[3.2.0]heptane ;
l'atome d'azote de l'amine cyclique Z pouvant être sous forme N-oxyde ;
les atomes de carbone de l'amine cyclique Z étant éventuellement substitués par un groupe $R_{12}$ ;
$R_{12}$ représente un groupe $NR_1R_2$, $NR_3COOR_5$, hydroxyle ;
$R_1$ et $R_2$, représentent, indépendamment l'un de l'autre, un atome d'hydrogène ;
$R_3$ représente un atome d'hydrogène ;
$R_5$ représente un groupe $C_1$-$C_6$-alkyle.

Parmi les composés de formule générale (I) objets de l'invention, un dix-huitième sous-groupe de composés

est constitué par les composés pour lesquels Z représente une amine cyclique choisie parmi l'azétidine, la pyrrolidine, la pipéridine, la morpholine, l'azabicylo[3.1.0] hexane et l'azabicylo[3.2.0]heptane ; l'atome d'azote de l'amine cyclique Z pouvant être sous forme N-oxyde ; les atomes de carbone de l'amine cyclique Z étant éventuellement substitués par un groupe $R_{12}$ ; $R_{12}$ représente un groupe $NR_1R_2$, $NR_3COOR_5$, hydroxyle ; $R_1$ et $R_2$, représentent, indépendamment l'un de l'autre, un atome d'hydrogène ; $R_3$ représente un atome d'hydrogène ; $R_5$ représente un groupe tertiobutyle.

Parmi les composés de formule générale (I) objets de l'invention, un dix-neuvième sous-groupe de composés est constitué par les composés pour lesquels Z représente une amine cyclique choisie parmi l'azétidine, la pyrrolidine, la pipéridine, la morpholine, l'azabicylo[3.1.0] hexane et l'azabicylo[3.2.0]heptane ; les atomes de carbone de l'azétidine étant éventuellement substitués par un groupe hydroxyle ; les atomes de carbone de la pyrrolidine étant éventuellement substitués par un groupe $NR_1R_2$, $NR_3COOR_5$, hydroxyle ; l'atome d'azote de la pyrrolidine pouvant être sous forme N-oxyde ; $R_1$ et $R_2$, représentent, indépendamment l'un de l'autre, un atome d'hydrogène ; $R_3$ représente un atome d'hydrogène ; $R_5$ représente un groupe $C_1$-$C_6$-alkyle.

Parmi les composés de formule générale (I) objets de l'invention, un vingtième sous-groupe de composés est constitué par les composés pour lesquels Z représente une amine cyclique choisie parmi l'azétidine, la pyrrolidine, la pipéridine, la morpholine, l'azabicylo[3.1.0] hexane et l'azabicylo[3.2.0]heptane ; les atomes de carbone de l'azétidine étant éventuellement substitués par un groupe hydroxyle ; les atomes de carbone de la pyrrolidine étant éventuellement substitués par un groupe $NR_1R_2$, $NR_3COOR_5$, hydroxyle ; l'atome d'azote de la pyrrolidine pouvant être sous forme N-oxyde ; $R_1$ et $R_2$, représentent, indépendamment l'un de l'autre, un atome d'hydrogène ; $R_3$ représente un atome d'hydrogène ; $R_5$ représente un groupe tertiobutyle.

Parmi les composés de formule générale (I) objets de l'invention, un vingt-et-unième sous-groupe de composés est constitué par les composés pour lesquels Z représente une amine cyclique reliée par l'atome d'azote, de formule :

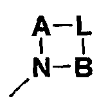

dans laquelle

- A représente un groupe $C_1$-$C_7$-alkylène éventuellement substitué par un ou deux groupes $R_8$ ;
- B représente un groupe $C_1$-$C_7$-alkylène éventuellement substitué par un ou deux groupes $R_9$ ;
- L représente une liaison, un atome de soufre, d'oxygène ou d'azote ;
- l'atome d'azote étant éventuellement substitué par un groupe $R_{10}$ ou $R_{11}$.

les atomes de carbone de l'amine cyclique Z étant éventuellement substitués par un ou plusieurs groupes $R_{12}$ identiques ou différents l'un de l'autre, $R_{12}$ représentant un groupe $C_1$-$C_6$-alkyle éventuellement substitué par un groupe $R_{13}$, $NR_1R_2$, $NR_3COOR_5$ ; $R_1$ et $R_2$, représentent, indépendamment l'un de l'autre, un atome d'hydrogène ou un groupe $C_1$-$C_6$-alkyle ; $R_3$ représente un atome d'hydrogène ou un groupe $C_1$-$C_6$-alkyle ; $R_5$ représente un groupe $C_1$-$C_6$-alkyle ; $R_8$, $R_9$ et $R_{10}$ sont définis tels que :

- deux groupes $R_8$ peuvent former ensemble une liaison ou un groupe $C_1$-$C_6$-alkylène ; ou
- deux groupes $R_9$ peuvent former ensemble une liaison ou un groupe $C_1$-$C_6$-alkylène ; ou
- $R_8$ et $R_9$ peuvent former ensemble une liaison ou un groupe $C_1$-$C_6$-alkylène ; ou
- $R_8$ et $R_{10}$ peuvent former ensemble une liaison ou un groupe $C_1$-$C_6$-alkylène ; ou

$R_9$ et $R_{10}$ peuvent former ensemble une liaison ou un groupe $C_1$-$C_6$-alkylène ;

$R_{11}$ étant tel que défini dans la formule générale (I).

Parmi les composés de formule générale (I) objets de l'invention, un vingt-deuxième sous-groupe de composés est constitué par les composés pour lesquels

Z représente une amine cyclique reliée par l'atome d'azote, de formule :

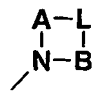

dans laquelle

A représente un groupe $C_1$-$C_7$-alkylène ;
B représente un groupe $C_1$-$C_7$-alkylène ;
L représente une liaison, un atome de soufre, d'oxygène ou d'azote ; l'atome d'azote étant éventuellement substitué par un groupe $R_{11}$.

les atomes de carbone de l'amine cyclique Z étant éventuellement substitués par un ou plusieurs groupes $R_{12}$ identiques ou différents l'un de l'autre ;

$R_{11}$ et $R_{12}$ étant tels que définis dans la formule générale (I).

Parmi les composés de formule générale (I) objets de l'invention, un vingt-troisième sous-groupe de composés est constitué par les composés pour lesquels Z représente un groupe azétidinyle, pyrrolidinyle, pipéridinyle, azépinyle, morpholinyle, thiomorpholinyle, pipérazinyle, homopipérazinyle, ce groupe étant éventuellement substitué par un ou plusieurs groupes $R_{12}$ identiques ou différents l'un de l'autre ; $R_{12}$ étant tel que défini dans la formule générale (I).

Parmi les composés de formule générale (I) objets de l'invention, un vingt-quatrième sous-groupe de composés est constitué par les composés pour lesquels Z représente un groupe pyrrolidinyle, pipéridinyle, morpholinyle, pipérazinyle, ce groupe étant éventuellement substitué par un ou plusieurs groupes $R_{12}$ identiques ou différents l'un de l'autre ;

$R_{12}$ représentant un groupe $C_1$-$C_6$-alkyle éventuellement substitué par un groupe

$R_{13}$, $NR_1R_2$, $NR_3COOR_5$;

$R_1$ et $R_2$, représentent, indépendamment l'un de l'autre, un atome d'hydrogène ou un groupe $C_1$-$C_6$-alkyle ;

$R_3$ représente un atome d'hydrogène ou un groupe $C_1$-$C_6$-alkyle ;

$R_5$ représente un groupe $C_1$-$C_6$-alkyle.

Parmi les composés de formule générale (I) objets de l'invention, un vingt-cinquième sous-groupe de composés est constitué par les composés pour lesquels $X_1$, $X_2$, $X_3$, $X_4$, $Z_1$, $Z_2$, $Z_3$, $Z_4$, n, Y et Z sont tels que définis dans les sous-groupes ci-dessus.

Parmi les composés de formule générale (I) objets de l'invention, un vingt-sixième sous-groupe de composés est constitué par les composés pour lesquels

$X_1$, $X_2$, $X_3$ et $X_4$ sont choisis, indépendamment l'un de l'autre, parmi un atome d'hydrogène ou d'halogène ou un groupe $C_1$-$C_6$-alkyle ou $C_1$-$C_6$-fluoroalkyle ; et/ou

$Z_1$, $Z_2$, $Z_3$, $Z_4$ représentent, indépendamment l'un de l'autre, un atome d'azote ou un groupe $C(R_6)$, l'un correspondant à un atome d'azote et les autres correspondant à un groupe $C(R_6)$ ; l'atome d'azote présent dans le cycle, défini comme azote de position 1, étant éventuellement substitué par $R_7$ lorsque l'atome de carbone en position 2 ou 4 par rapport à cet azote de référence est substituté par un groupe oxo ou thio ; $R_6$ et $R_7$ étant tels que définis dans la formule générale (I) ; et/ou

n est égal à 1 ; et/ou

Z représente une amine cyclique reliée par l'atome d'azote, de formule :

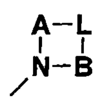

dans laquelle

A représente un groupe $C_1$-$C_7$-alkylène ;
B représente un groupe $C_1$-$C_7$-alkylène ;

L représente une liaison, un atome de soufre, d'oxygène ou d'azote ; l'atome d'azote étant éventuellement substitué par un groupe $R_{11}$.

les atomes de carbone de l'amine cyclique Z étant éventuellement substitués par un ou plusieurs groupes $R_{12}$ identiques ou différents l'un de l'autre ;
$R_{11}$ et $R_{12}$ étant tels que définis dans la formule générale (I).

Parmi les composés de formule générale (I) objets de l'invention, on peut citer les composés suivants :

1. *N*-{6-[((*R*)-3-terbutoxycarbonylamino)-pyrrolidin-1-yl]-pyridin-3-yl}-5-fluoro-1-(3-fluorobenzyl)-1*H*-indole-2-carboxamide

2. *N*-[6-(pyrrolidin-1-yl)-pyridin-3-yl]-5-fluoro-1-(3-fluorobenzyl)-1*H*-indole-2-carboxamide

3. *N*-{6-[(*R*)-3-aminopyrrolidin-1-yl]-pyridin-3-yl}-5-fluoro-1-(3-fluorobenzyl)-1*H*-indole-2-carboxamide

4. *N*-[6-(azétidin-1-yl)-pyridin-3-yl]-5-fluoro-1-(3-fluorobenzyl)-1*H*-indole-2-carboxamide

5. *N*-[6-(pyrrolidin-1-yl)-pyridin-3-yl]-5-trifluorométhyl-1-(3-fluorobenzyl)-1*H*-indole-2-carboxamide

6. *N*-[4-mèthyl-6-(pyrrolidin-1-yl)-pyridin-3-yl]-5-fluoro-1-(3-fluorobenzyl)-1*H*-indoie-2-carboxamide

7. *N*-[6-(3-hydroxypyrrolidin-1-yl)-pyridin-3-yl]-5-fluoro-1-(3-fluorobenzyl)-1*H*-indole-2-carboxamide

8. *N*-[5-méthoxy-6-(pyrrolidin-1-yl)-pyridin-3-yl]-5-fluoro-1-(3-fluorobenzyl)-1*H*-indole-2-carboxamide

9. *N*-[6-(pyrrolidin-1-yl)pyridin-3-yl]-1-[(pyridin-4-yl)méthyl]-6-trifluorométhyl-1*H*-indole-2-carboxamide

10. *N*-[6-(pyrrolidin-1-yl)-4-méthylpyridin-3-yl]-5-fluoro-1-[(pyridin-4-yl)méthyl]-1*H*-indole-2-carboxamide

11. *N*-[5-méthoxy-6-(pyrrolidin-1-yl)-pyridin-3-yl]-5-fluoro-1-[(pyridin-4-yl)méthyl]-1*H*-indole-2-carboxamide

12. *N*-[6-(pyrrolidin-1-yl)-pyridin-3-yl]-5-fluoro-1-[3-(pyrrolidin-1-yl)benzyl]-1*H*-indole-2-carboxamide

13. *N*-[6-(piperidin-1-yl)-pyridin-3-yl]-5-fluoro-1-(3-fluorobenzyl)-1*H*-indole-2-carboxamide

14. *N*-[6-(pyrrolidin-1-yl)pyridin-3-yl]-1-[(pyridin-4-yl)méthyl]-5-trifluorométhyl-1*H*-indole-2-carboxamide

15. *N*-[6-(pyrrolidin-1-yl)pyridin-3-yl]-5-fluoro-1-[(6-méthylpyridin-2-yl)méthyl]-1*H*-indole-2-carboxamide

16. *N*-[4-méthoxy-6-(pyrrolidin-1-yl)-pyridin-3-yl]-5-fluoro-1-(3-fluorobenzyl)-1*H*-indole-2-carboxamide

17. Chlorhydrate (1:1) de *N*-[6-(morpholin-4-yl)-pyridin-3-yl]-5-fluoro-1-(3-fluorobenzyl)-1*H*-indole-2-carboxamide

18. *N*-[4-méthoxy-6-(pyrrolidin-1-yl)-pyridin-3-yl]-5-fluoro-1-[(pyridin-4-yl)méthyl]-1*H*-indole-2-carboxamide

19. *N*-[6-(pyrrolidin-1-yl)pyridin-3-yl]-5-fluoro-1-[(2-méthylpyridin-4-yl)méthyl]-1*H*-indole-2-carboxamide

20. *N*-[6-(1-oxy-pyrrolidin-1-yl)-pyridin-3-yl]-5-fluoro-1-(3-fluorobenzyl)-1*H*-indole-2-carboxamide

21. *N*-[2-(pyrrolidin-1-yl)-pyrimidin-5-yl]-5-fluoro-1-(3-fluorobenzyl)-1*H*-indole-2-carboxamide

22. *N*-[6-(3-aza-bicyclo[3.2.0]hept-3-yl)-pyridin-3-yl]-5-fluoro-1-(3-fluorobenzyl)-1*H*-indole-2-carboxamide

23. *N*-[6-(pyrrolidin-1-yl)pyridin-3-yl]-1-[(pyridin-4-yl)méthyl]-5-fluoro-1*H*-indole-2-carboxamide

24. *N*-[6-(3-hydroxyazétidin-1-yl)-pyridin-3-yl]-5-fluoro-1-(3-fluorobenzyl)-1*H*-indole-2-carboxamide

25. *N*-[6-(azétidin-1-yl)-pyridin-3-yl]-5-trifluorométhyl-1-(3-fluorobenzyl)-1*H*-indole-2-carboxamide

26. *N*-[6-(azétidin-1-yl)pyridin-3-yl]-1-[(pyridin-4-yl)méthyl]-5-fluoro-1*H*-indole-2-carboxamide

27. *N*-[2-méthyl-6-(pyrrolidin-1-yl)-pyridin-3-yl]-5-fluoro-1-(3-fluorobenzyl)-1*H*-indole-2-carboxamide

28. *N*-[6-(3-aza-bicyclo[3.1.0]hex-3-yl)-pyridin-3-yl]-5-fluoro-1-(3-fluorobenzyl)-1*H*-indole-2-carboxamide

29. *N*-[6-(3-hydroxyazétidin-1-yl)-pyridin-3-yl]-1-(3-fluorobenzyl)-5-trifluorométhyl-1*H*-indole-2-carboxamide

30. *N*-[6-(3-hydroxyazétidin-1-yl)-pyridin-3-yl]-1-(3-fluorobenzyl)-6-trifluorométhyl-1*H*-indole-2-carboxamide

31. *N*-[6-(azétidin-1-yl)-pyridin-3-yl]-5-fluoro-1-(benzyl)-1*H*-indole-2-carboxamide

32. *N*-[6-(azétidin-1-yl)-pyridin-3-yl]-4,6-diméthyl-1-(3-fluorobenzyl)-1*H*-indole-2-carboxamide

33. *N*-[6-(azétidin-1-yl)-pyridin-3-yl]-6-terbutyl-1-(3-fluorobenzyl)-1*H*-indole-2-carboxamide

34. *N*-[6-(azétidin-1-yl)-pyridin-3-yl]-6-isopropyloxy-1-(3-fluorobenzyl)-1*H*-indole-2-carboxamide

35. *N*-[6-(azétidin-1-yl)-pyridin-3-yl]-5-méthylsulfonyl-1-(3-fluorobenzyl)-1*H*-indole-2-carboxamide

36. *N*-[6-(azétidin-1-yl)-pyridin-3-yl]-6-diméthylamino-1-(3-fluorobenzyl)-1*H*-indole-2-carboxamide

37. *N*-[6-(azétidin-1-yl)-pyridin-3-yl]-6-méthylthio-1-(3-fluorobenzyl)-1*H*-indole-2-carboxamide

38. *N*-[6-(azétidin-1-yl)-pyridin-3-yl]-4-fluoro-1-(3-fluorobenzyl)-1*H*-indole-2-carboxamide

39. *N*-[6-(azétidin-1-yl)-pyridin-3-yl]-7-fluoro-1-(3-fluorobenzyl)-1*H*-indole-2-carboxamide

40. *N*-[6-(azétidin-1-yl)-pyridin-3-yl]-6-fluoro-1-(3-fluorobenzyl)-1*H*-indole-2-carboxamide

41. *N*-[6-(azétidin-1-yl)-pyridin-3-yl]-4-chloro-1-(3-fluorobenzyl)-1*H*-indole-2-carboxamide

42. *N*-[6-(azétidin-1-yl)-pyridin-3-yl]-6-chloro-1-(3-fluorobenzyl)-1*H*-indole-2-carboxamide

43. *N*-[6-(azétidin-1-yl)-pyridin-3-yl]-5-chloro-1-(3-fluorobenzyl)-1*H*-indole-2-carboxamide

44. *N*-[6-(azétidin-1-yl)-pyridin-3-yl]-1-(3-fluorobenzyl)-1*H*-indole-2-carboxamide

45. *N*-[6-(azétidin-1-yl)-pyridin-3-yl]-4-méthyl-1-(3-fluorobenzyl)-1*H*-indole-2-carboxamide

46. *N*-[6-(azétidin-1-yl)-pyridin-3-yl]-6-méthyl-1-(3-fluorobenzyl)-1*H*-indole-2-carboxamide

47. *N*-[6-(azétidin-1-yl)-pyridin-3-yl]-5-méthyl-1-(3-fluorobenzyl)-1*H*-indole-2-carboxamide

48. *N*-[6-(azétidin-1-yl)-pyridin-3-yl]-5-isopropyl-1-(3-fluorobenzyl)-1*H*-indole-2-carboxamide

49. *N*-[6-(azétidin-1-yl)-pyridin-3-yl]-5-terbutyl-1-(3-fluorobenzyl)-1*H*-indole-2-carboxamide

50. *N*-[6-(azétidin-1-yl)pyridin-3-yl]-6-éthyl-1-(3-fluorobenzyl)-1*H*-indole-2-carboxamide

51. *N*-[6-(azétidin-1-yl)-pyridin-3-yl]-6-isopropyl-1-(3-fluorobenzyl)-1*H*-indole-2-carboxamide

52. *N*-[6-(azétidin-1-yl)-pyridin-3-yl]-6-trifluorométhyl-1-(3-fluorobenzyl)-1*H*-indole-2-carboxamide

53. *N*-[6-(pyrrolidin-1-yl)-4-(trifluorométhyl)pyridin-3-yl]-5-fluoro-1-(3-fluorobenzyl)-1*H*-indole-2-carboxamide

Dans le cadre de la présente invention on entend par :

- $C_t$-$C_z$ où t et z peuvent prendre les valeurs de 1 à 7, une chaîne carbonée pouvant avoir de t à z atomes de carbone, par exemple $C_1$-$C_3$ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe $C_{1-3}$-alkylène représente une chaîne carbonée divalente de 1 à 3 atomes de carbone, linéaire ou ramifiée, par exemple un méthylène, éthylène, 1-méthyléthylène, propylène ;
- un cycloalkyle : un groupe carboné cyclique. A titre d'exemples, on peut citer les groupes cyclopropyle, cyclobutyle, cyclopentyle, cyclohexyle, etc ;
- un hétérocycloalkyle : un groupe cyclique de 3 à 7 chaînons contenant de 1 à 2 hétéroatomes choisis parmi O, S ou N ;
- un cycloalk-1,1-diyle ou un hétérocycloalk-1,1-diyle : un groupe du type

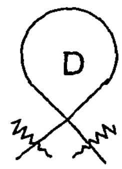

  où D est un groupe cycloalkyle ou hétérocycloalkyle ;
- un fluoroalkyle : un groupe alkyle dont un ou plusieurs atomes d'hydrogène ont été substitués par un atome de fluor ;
- un alcoxyle : un radical -O-alkyle où le groupe alkyle est tel que précédemment défini ;
- un cycloalcoxyle : un radical -O-cycloalkyle où le groupe cylcoalkyle est tel que précédemment défini ;
- un fluoroalcoxyle : un groupe alcoxyle dont un ou plusieurs atomes d'hydrogène ont été substitués par un atome de fluor ;
- un thioalkyle : un radical -S-alkyle où le groupe alkyle est tel que précédemment défini ;
- un aryle : un groupe aromatique cyclique comprenant entre 6 et 10 atomes de carbones. A titre d'exemples de groupes aryles, on peut citer les groupes phényle ou naphtyle ;
- un hétéroaryle : un groupe cyclique aromatique de 5 à 10 chaînons contenant de 1 à 4 hétéroatomes choisis parmi O, S ou N. A titre d'exemple, on peut citer les groupes imidazolyle, thiazolyle, oxazolyle, furanyle, thiophényle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle, quinoxalinyle ;
- un atome d'halogène : un fluor, un chlore, un brome ou un iode ;
- « oxo » signifie « = O » ;
- « thio » signifie « = S ».

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

Conformément à l'invention on peut préparer les composés de formule générale (I) selon le procédé illustré par le schéma 1 qui suit.

Selon le schéma 1, les composés de formule générale (IV) peuvent être obtenus par réaction d'un composé de formule générale (II) dans laquelle $X_1$, $X_2$, $X_3$, $X_4$ sont tels que définis dans la formule générale (I) et B représente

un groupe $C_1$-$C_6$-alcoxyle, avec un composé de formule générale (III), dans laquelle Y et n sont tels que définis dans la formule générale (I) et GP représente un groupe partant où GP représente un groupe hydroxyle.

Les composés de formule générale (II) sont disponibles dans le commerce ou préparés selon de nombreux procédés décrits dans la littérature (D. Knittel Synthesis 1985, 2, 186 ; T.M. Williams J.Med.Chem. 1993, 36 (9), 1291 ; JP2001151771A2 par exemple).

Lorsque le composé de formule générale (III), est défini tel que n est égal à 1, 2 ou 3 et GP représente un groupe partant tel qu'un atome de chlore, de brome ou d'iode, la réaction peut être réalisée en présence d'une base telle que l'hydrure de sodium ou le carbonate de potassium, dans un solvant polaire tel que le diméthylformamide, le diméthylsulfoxyde ou l'acétone (n = 1 : Kolasa T., Bioorg.Med.Chem. 1997, 5 (3) 507, n = 2 : Abramovitch R., Synth. Commun., 1995, 25 (1), 1).

Lorsque le composé de formule générale (III) est défini tel que n est égal à 1, 2 ou 3 et GP représente un groupe hydroxyle, les composés de formule générale (IV), peuvent être obtenus par réaction du composé de formule générale (II) avec un composé de formule générale (III) en présence d'une phosphine telle que, par exemple, la triphénylphosphine et d'un réactif tel que, par exemple, l'azodicarboxylate de diéthyle en solution dans un solvant tel que le dichlorométhane ou le tétrahydrofuranne (O. Mitsonobu, Synthesis, 1981, 1-28).

Schéma 1

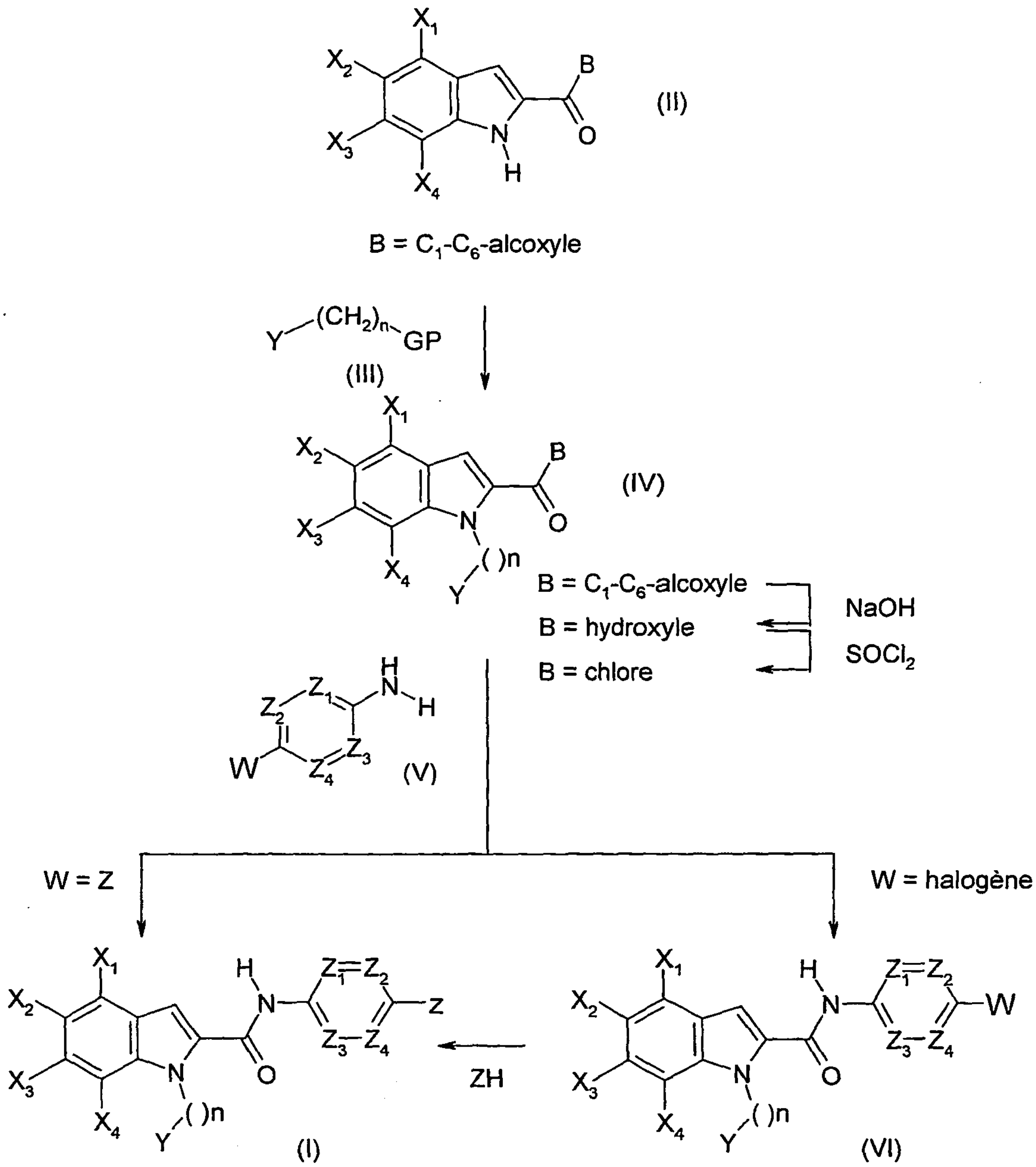


Lorsque le composé de formule générale (III) est défini tel que n est égal à 0, GP représente un groupe partant tel qu'un atome de chlore, de brome ou d'iode et la réaction peut être réalisée à une température comprise entre 80°C et 250°C, en présence d'un catalyseur à base de cuivre tel que le bromure de cuivre ou l'oxyde de cuivre ainsi que d'une base telle que le carbonate de potassium (Murakami Y., Chem.Pharm.Bull., 1995, 43 (8), 1281). On peut également utiliser les conditions plus douces, décrites dans S.L. Buchwald, J.Am.Chem.Soc. 2002, 124, 11684.

Le composé de formule générale (IV), pour lequel B représente un groupe $C_1$-$C_6$-alcoxyle, peut être transformé en composé de formule générale (IV), où B représente un groupe hydroxyle, par l'action d'une base telle que la soude ou la potasse en solution dans un solvant tel que l'éthanol. Le composé de formule générale (IV), où B représente un groupe hydroxyle peut, par la suite, être transformé en composé de formule générale (IV), où B représente un atome de chlore, par l'action d'un agent chlorant tel que que le chlorure de thionyle dans un solvant tel que le dichlorométhane.

Les composés de formule générale (I) et (VI) peuvent ensuite être obtenus, par exemple, par réaction d'un composé de formule générale (IV) où B est un atome de chlore, tel qu'obtenu ci-dessus, avec une amine de formule générale (V), dans laquelle $Z_1$, $Z_2$, $Z_3$, $Z_4$ sont tels que définis dans la formule générale (I) et W représente un atome

d'halogène, tel qu'un chlore, un brome ou un iodure, ou à une amine cyclique Z telle que définie dans la formule générale de (I), dans un solvant tel que le dichloroéthane, le toluène ou le tétrahydrofuranne.

Les composés de formule générale (I) et (VI) peuvent également être obtenus par réaction d'un composé de formule générale (IV) où B est un groupe hydroxyle, tel qu'obtenu ci-dessus, avec une amine de formule générale (V), dans laquelle $Z_1$, $Z_2$, $Z_3$ et $Z_4$ sont tels que définis dans la formule générale (I) et W représente un atome d' halogène, tel qu'un chlore, un brome ou un iodure, ou à une amine cyclique Z tel que défini dans la formule générale de (I), en présence d'un agent de couplage tel que le diéthylcyanophosphonate, en présence d'une base telle que la triéthylamine, dans un solvant tel que le diméthylformamide, ou en présence d'un agent de couplage tel que le *N*-(3-diméthylaminopropyl)-*N*'-éthylcarbodiimide, en présence de *N*-1-hydroxybenzotriazole, dans un solvant tel que le diméthylformamide.

Dans le cas où W représente un atome d'halogène, on obtient un composé de formule générale (VI). Dans le cas où W représente une amine cyclique Z telle que définie dans la formule générale de (I), on obtient un composé de formule générale (I).

Le composé de formule générale (I) peut également être obtenu par réaction d'un composé de formule générale (VI) où W représente un atome d'halogène, en présence d'une amine de formule ZH dans laquelle Z est une aminé cyclique telle que définie dans la formule générale (I), sans solvant ou dans un solvant tel que la N-méthylpyrrolidinone, ou bien par application d'une méthode de N-arylation catalysée telle que celle décrite par J. Hartwig (J.F. Hartwig, Angew Chem. Int. Ed. 2005, 44, 1371-1375), de préférence sous atmosphère inerte, en présence d'une base telle le bis-triméthylsilylamidure de lithium en présence d'une source de palladium en quantité catalytique, tel que le diacétate de palladium, et d'une quantité catalytique d'un ligand du palladium, telle qu'une phosphine, le tout en solution dans un solvant tel que le diméthoxyéthane (DME).

Les composés de formules générales (I), (II) et (IV), dans lesquelles $X_1$, $X_2$, $X_3$ et/ou $X_4$, représentent un groupe cyano ou un aryle, peuvent être obtenus par une réaction de couplage, catalysée par un métal tel que le palladium, réalisée sur les composés de formules générales (I), (II) et (IV) correspondants, dans lesquelles $X_1$, $X_2$, $X_3$ et /ou $X_4$, représentent un groupe partant, par exemple un brome, selon des méthodes qui sont décrites dans la littérature ou qui sont connues de l'homme de métier.

Les composés de formules générales (I), (II) et (IV), dans lesquelles $X_1$, $X_2$, $X_3$ et/ou $X_4$, représentent un groupe C(O)$NR_1R_2$, peuvent être obtenus à partir des composés de formules générales (I), (II) et (IV) correspondants, dans lesquelles $X_1$, $X_2$, $X_3$ et/ou $X_4$, représentent un groupe cyano, selon des méthodes qui sont décrites dans la littérature ou qui sont connues de l'homme du métier.

Les composés de formules générales (I), (II) et (IV), dans lesquelles $X_1$, $X_2$, $X_3$, $X_4$ et/ou $R_6$ correspondent à un groupe -S(O)-alkyle ou -S(O)$_2$-alkyle, peuvent être obtenus par oxydation des composés de formules générales (I), (II) et (IV) correspondants, dans lesquelles $X_1$, $X_2$, $X_3$, $X_4$ et/ou $R_6$ représentent un groupe $C_1$-$C_6$-thioalkyle, selon des méthodes qui sont décrites dans la littérature ou qui sont connues de l'homme du métier.

De même, les composés de formules générales (I) et (IV), dans lesquelles Y est substitué par un groupe -S(O)-alkyle ou -S(O)$_2$-alkyle, peuvent être obtenus par oxydation des composés de formules générales (I) et (IV) correspondants, dans lesquelles Y est substitué par un groupe $C_1$-$C_6$-thioalkyle, selon des méthodes qui sont décrites dans la littérature ou qui sont connues de l'homme du métier.

Les composés de formule générale (I) dans lesquelles $Z_1$, $Z_2$, $Z_3$ et/ou $Z_4$ représentent un groupe C-$R_6$ correspondant à un groupe C-OH, peuvent être obtenus au départ des composés de formule générale (I) correspondants, dans lesquelles $Z_1$, $Z_2$, $Z_3$ et/ou $Z_4$ représentent un groupe C-$R_6$ correspondant à un groupe $C_1$-$C_6$-alcoxyle, selon des méthodes qui sont décrites dans la littérature ou qui sont connues de l'homme du métier.

Les composés de formules générales (1), (II) et (IV), dans lesquelles $X_1$, $X_2$, $X_3$ et/ou $X_4$, représentent un groupe $NR_1R_2$, $NR_3COR_4$ ou $NR_3SO_2R_5$, peuvent être obtenus à partir des composés de formules générales(I), (II) et (IV) correspondants, dans lesquelles $X_1$, $X_2$, $X_3$, et/ou $X_4$, représentent un groupe nitro, par exemple par réduction, puis acylation ou sulfonylation, selon des méthodes qui sont décrites dans la littérature ou qui sont connues de l'homme du métier.

Les composés de formules générales (I), (II) et (IV), dans lesquelles $X_1$, $X_2$, $X_3$ et/ou $X_4$, représentent un groupe $NR_1R_2$, $NR_3COR_4$ ou $NR_3SO_2R_5$, peuvent être obtenus à partir des composés de formules générales (I), (II) et (IV) correspondants, dans lesquelles $X_1$, $X_2$, $X_3$, et/ou $X_4$, représentent, par exemple, un atome de brome par réaction de couplage respectivement avec une amine, un amide ou une sulfonamide en présence d'une base, d'une phosphine et d'un catalyseur à base de palladium, selon des méthodes qui sont décrites dans la littérature ou qui sont connues de l'homme du métier.

Les composés de formules générales (I), (II) et (IV), dans lesquelles $X_1$, $X_2$, $X_3$ et/ou $X_4$, représentent un groupe $SO_2NR_1R_2$ peuvent être obtenus par une méthode analogue à celle décrite dans Pharmazie 1990, 45, 346, ou selon des méthodes qui sont décrites dans la littérature ou qui sont connues de l'homme du métier.

Les composés de formule générale (III) sont disponibles dans le commerce, décrits dans la littérature (Carling R.W. et al J.Med.Chem. 2004 (47), 1807 - 1822 ou Russel M.G.N. et al. J. Med. Chem. 2005 (48), 1367 - 1383) ou accessibles en utilisant des méthodes connues de l'homme du métier. Certains composés de formule générale (IV) sont décrits

dans la littérature (WO07/010144, par exemple). Les composés (V) et les autres réactifs, quand leur mode de préparation n'est pas décrit, sont disponibles dans le commerce ou décrits dans la littérature (WO05028452, WO02048152, WO06040522, WO04052869, Heterocycles 1977, 6(12), 1999 - 2004, J.Chem.Soc. Perkin trans 1, 1973 (1) 68-69, JP07/051121, WO05035526, WO07/011284, WO04/062665, GB870027, US4104385).

L'invention, selon un autre de ses aspects, a également pour objet les composés de formules (Va), (Vb), (Vc), (Vd), (Vf), (Vg). Ces composés sont utiles comme intermédiaires de synthèse des composés de formule (I).

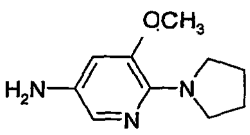
(Va)

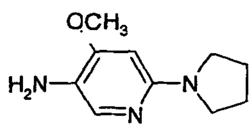
(Vb)

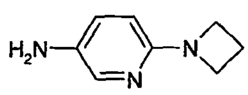
(Vc)

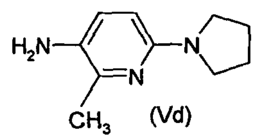
(Vd)

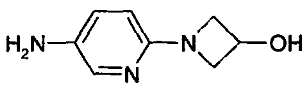
(Ve)

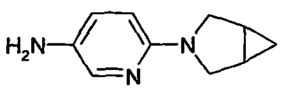
(Vf)

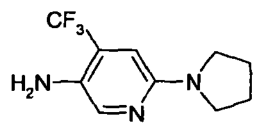
(Vg)

Les amines de formules (Va), (Vb), (Vc), (Vd), (Ve), (Vf), (Vg) peuvent être préparées, par exemple selon le procédé décrit dans l'exemple n°5, par substitution nucléophile aromatique d'un précurseur 6-chloropyridine, éventuellement substitué (Va : par un groupe 5-méthoxy, Vb : par un groupe 4-méthoxy, Vd : par un groupe 2-méthyl, Vg : par un groupe 4-trifluorométhyl) avec une amine, telle que la pyrrolidine, par exemple dans un solvant tel que l'éthanol. L'accès aux amines Va-g peut ensuite nécessiter la réduction d'un groupe nitro, par exemple, par hydrogénation catalytique en présence d'un catalyseur tel que le palladium sur charbon, ou par toutes autres méthodes connues de l'homme de l'art, de réduction d'un groupe nitro en amine. Les amines de formules (Va), (Vb), (Vc), (Vd), (Ve), (Vf), (Vg) ont été préparées à l'état de poudre ou d'huile, à l'état de base ou de sel d'addition à un acide. Le tableau 1 rassemble les données RMN $^1H$ de ces amines.

Tableau 1

| N° | R.M.N. $^1H$, δ (ppm): |
|---|---|
| Va | Dans le $CDCl_3$, δ (ppm): 2,5 (m, 4H) ; 3,23 (pic élargi, 2H) ; 3,49 (m, 4H) ; 3,91 (s, 3H) ; 5,90 (s, 1H) ; 7,71 (s, 1H). |
| Vb | Dans le DMSO $D_6$, δ (ppm): 1,81 (m, 4H) ; 3,29 (m, 4H) ; 3,71 (s, 3H) ; 4,52 (pic élargi, 2H) ; 6,61 (s, 1H) ; 7,16 (s, 1H). |
| Vc | Dans le DMSO $D_6$, δ (ppm): 2,21 (m, 2H) ; 3,78 (m, 4H) ; 4,42 (pic élargi, 2H) ; 6,20 (d, 1H) ; 6,89 (d, 1H) ; 7,58 (s, 1H). |
| Vd | Dans le $CDCl_3$, δ (ppm): 1.89 (m, 4H) ; 2,23 (s, 3H) ; 2,98 (pic élargi, 2H) ; 3,31 (m, 4H) ; 6,03 (d, 1H) ; 6,82 (d, 1H). |
| Ve | Dans le DMSO $D_6$, δ (ppm): 3,49 (m, 2H) ; 3,98 (m, 2H) ; 4,48 (pic élargi, 3H) ; 5,48 (pic élargi, 1H) ; 6,22 (d, 1H) ; 6,91 (d, 1H) ; 7,58 (s, 1H). |
| Vf | Dans le DMSO $D_6$, δ (ppm): 0,2 (m, 1H) ; 0,66 (m, 1H) ; 1,6 (m, 2H) ; 3,11 (m, 2H) ; 3,51 (d, 2H) ; 4,32 (pic élargi, 2H) ; 6,25 (d, 1H) ; 6,99 (d,1H) ; 7,55 (s, 1H). |
| Vg | Dans le DMSO $D_6$, δ (ppm): 2 (m, 4H) ; 3,52 (m, 4H) ; 7,1 (s, 1H) ; 7,49 (pic élargi, 2H) ; 7,92 (s, 1H); forme chlorydrate 1:1 |

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 2. Les microanalyses élémentaires, les analyses LC-MS (chromatographie liquide couplée à la spectrométrie de masse) les spectres I.R. ou les spectres R.M.N. confirment les structures des composés obtenus.

**<u>Exemple 1</u> (Composé N°1)**

*N*-{6-[((*R*)-3-terbutoxycarbonylamino)-pyrrolidin-1-yl]-pyridin-3-yl}-5-fluoro-1-(3-fluorobenzyl)-1*H*-indole-2-carboxamide

1.1. Acide 5-fluoro-1-(3-fluorobenzyl)-1*H*-indole-2-carboxylique

On ajoute une solution aqueuse de soude, préparée à partir de 1,15 g (28,92 mmoles) de pastille de soude dans 50 mL d'eau, à une solution de 7,6 g (24,10 mmoles) de 5-fluoro-1-(3-fluorobenzyl)-1*H*-indole-2-carboxylate d'éthyle (WO2006/024776) dans 241 mL d'éthanol. Le mélange est chauffé durant deux heures puis concentré sous pression réduite. Le solide résultant est repris dans 200 mL d'eau. La solution est lavée avec deux fois 100 mL d'éther éthylique, acidifiée par ajouts successifs de petites quantités d'acide chlorhydrique concentré puis extraite avec 200 mL d'acétate d'éthyle. La phase organique est finalement lavée deux fois avec 100 mL d'eau, une fois avec 50 ml d'une solution saturée en chlorure de sodium, séchée sur sulfate de magnésium et concentrée sous pression réduite. On obtient, après séchage à 50°C sous pression réduite, 6,4 g du produit attendu sous la forme d'un solide qui sera utilisé tel quel dans l'étape suivante.

1.2 *N*-{6-[((*R*)-3-terbutoxycarbonylamino)pyrrolidin-1-yl]pyridin-3-yl}-5-fluoro-1-(3-fluorobenzyl)-1*H*-indole-2-carboxamide (composé n°1)

On ajoute goutte à goutte, à 20°C sous argon, à une solution de 0,47 g (1,64 mmole) d'acide 5-fluoro-1-(3-fluorobenzyl)-1*H*-indole-2-carboxylique (obtenu à l'étape 1.1), de 0,5 g (1,8 mmole) de 3-amino-6-[((*R*)-3-terbutoxycarbonylamino)-pyrrolidin-1-yl]pyridine (JP2004175739) et de 0,85 g (1,64 mmole) de tris-pyrrolidino-phosphonium hexafluorophosphate (PYBOP) dans 20 mL de dichlorométhane, 0,85 mL (4,91 mmoles) de diispropyléthylamine. Le mélange est agité 12 heures à température ambiante, concentré sous pression réduite puis repris avec 50 mL d'acétate d'éthyle. Cette solution est alors, successivement lavée avec trois fois 15 mL d'une solution saturée en hydrogénocarbonate de sodium et 20 mL d'une solution saturée en chlorure de sodium puis séchée sur sulfate de sodium, filtrée et concentrée sous pression réduite. Le produit obtenu est purifié par chromatographie sur colonne de silice en éluant avec un mélange de dichlorométhane et de méthanol. Le solide résultant est ensuite trituré dans l'éther éthylique. On obtient 0,788 g d'un solide qui est séché sous pression réduite.
Point de fusion : 207 - 209 °C
R.M.N. $^1$H (DMSO $D_6$), $\delta$ (ppm): 1,49 (s, 9H) ; 1,85 (sext, 1H) ; 2,1 (sext, 1H) ; 3,2 (m, 4H) ; 4,09 (m, 1H) ; 5,88 (s, 2H) ; 6,41 (d, 1H) ; 7,04 (m, 7H) ; 7,61 (m, 2H) ; 7,8 (dxd, 1H) ; 8,31 (d, 1H) ; 10,19 (s, 1H).

**<u>Exemple 2</u> (Composé N°2)**

*N*-[6-(pyrrolidin-1-yl)-pyridin-3-yl]-5-fluoro-1-(3-fluorobenzyl)-1*H*-indole-2-carboxamide

On ajoute goutte à goutte, à 20°C sous argon, à une solution de 0,402 g (1,4 mmole) d'acide 5-fluoro-1-(3-fluorobenzyl)-1*H*-indole-2-carboxylique (exemple 1.1) et de 0,274 g (1,68 mmole) de 3-amino-6-(pyrrolidin-1-yl)-pyridine (WO02/48152) dans 10 mL de diméthylformamide sec, 0,28 mL (1,68 mmole) de diéthylcyanophosphonate. Le mélange est agité dix minutes puis on ajoute, au goutte à goutte, 0,43 mL (3,08 mmoles) de triéthylamine. Le mélange est agité 18 heures à température ambiante, concentré sous pression réduite puis repris avec 50 mL d'acétate d'éthyle. Cette solution est alors, successivement lavée avec trois fois 20 mL d'une solution saturée en hydrogénocarbonate de sodium, 50 mL d'eau et 20 mL d'une solution saturée en chlorure de sodium puis séchée sur sulfate de sodium, filtrée et concentrée sous pression réduite. Le solide obtenu est trituré dans l'éther isopropylique à chaud. On obtient 0,527 g d'un solide qui est séché sous pression réduite. Point de fusion : 199 - 201 °C
R.M.N. $^1$H (DMSO $D_6$), $\delta$ (ppm): 1,9 (m, 4H) ; 3,3 (m, 4H) ; 5,82 (s, 2H) ; 6,4 (d, 1H) ; 7,04 (m, 6H) ; 7,51 (m, 2H) ; 7,77 (dxd, 1H) ; 8,3 (d, 1H) ; 10,25 (s, 1H)

**<u>Exemple 3</u> (Composé N°4)**

*N*-[6-(azétidin-1-yl)-pyridin-3-yl]-5-fluoro-1-(3-fluorobenzyl)-1H-indole-2-carboxamide

3.1 *N*-[6-chloropyridin-3-yl]-5-fluoro-1-(3-fluorobenzyl)-1*H*-indole-2-carboxamide

On ajoute à une solution, agitée à 20°C sous atmosphere d'argon, de 4,48 g (34,89 mmoles) de 6-chloro-3-aminopyridine dans 317 mL de toluène, 23,8 mL (47,57 moles) d'une solution 2M de triméthylaluminium dans le toluène.

On porte ensuite la solution à 120˚C et on ajoute en plusieurs fois 10 g (31,71 mmoles) de 5-fluoro-1-(3-fluorobenzyl)-1H-indole-2-carboxylate d'éthyle (WO2006/024776). Le mélange réactionnel est chauffé 4 heures à reflux puis est refroidi à 0˚C. On additionne 15 mL d'eau au milieu réactionnel, que l'on extrait ensuite avec 2 fois 200 mL d'acétate d'éthyle. Les phases organiques sont réunies et lavées deux fois avec 50 mL d'eau et une fois avec 50 mL d'une solution saturée en chlorure de sodium puis séchées sur sulfate de magnésium et concentrées sous pression réduite. Le résidu obtenu est trituré dans 100 mL d'éther éthylique, on filtre et on recueille 10 g de produit attendu sous la forme d'une poudre.
R.M.N. $^1H$ (DMSO $D_6$), δ (ppm): 5,9 (s, 2H) ; 6,9 (m, 2H) ; 7,05 (txd, 1H) ; 7,2 (txd, 1H) ; 7,32 (m, 1H) ; 7,58 (m, 4H) ; 8,25 (d, 1H) ; 8,79 (s, 1H) ; 10,79 (s, 1H)

3.2 *N*-[6-(azétidin-1-yl)-pyridin-3-yl]-5-fluoro-1-(3-fluorobenzyl)-1*H*-indole-2-carboxamide (Composé n˚4)

A un mélange, agité à 0˚C sous atmosphere d'argon, de 0,7 g (1,76 mmole) de *N*-[6-chloropyridin-3-yl]-5-fluoro-1-(3-fluorobenzyl)-1*H*-indole-2-carboxamide, préparé à l'étape 3.1, de 0,123 g (2,11 mmoles) d'azétidine, de 8 mg (0,04 mmole) de palladium diacétate et de 19,5 mg (0,04 mmole) de (*R*)-(-)-1-[(*S*)-2-(dicyclohexylphosphino)ferrocenyl]éthylditerbutylphosphine dans 1,8 mL de diméthoxyéthane sec et dégazé sont additionnés 4,2 mL (4,2 mmoles) d'une solution de bis-triméthylsilylamidure de lithium 1 M dans le tétrahydrofurane. Le réacteur est fermé puis chauffé 16 heures à 100˚C. Le mélange réactionnel est ensuite versé dans 50 mL d'eau. On ajoute lentement 5 mL d'une solution molaire d'acide chlorhydrique puis 50 mL d'une solution saturée en hydrogénocarbonate de sodium. Le mélange est extrait deux fois avec 50 mL d'acétate d'éthyle. Les phases organiques sont réunies puis lavées deux fois par 50 mL d'une solution saturée en hydrogénocarbonate de sodium, séchées sur sulfate de magnésium puis concentrées sous pression réduite. Le produit obtenu est purifié par chromatographie sur colonne de silice en éluant avec un mélange de dichlorométhane et d'acétone. Le solide résultant est ensuite trituré dans 20 mL d'isopropanol chaud, recueilli par filtration puis recristallisé dans un mélange d'éthanol et de méthanol. On recueille par filtration 0,31 g d'un solide séché sous pression réduite.
Point de fusion : 227 - 228 ˚C
R.M.N. $^1H$ (DMSO $D_6$), δ (ppm): 2,25 (pent, 2H) ; 3,9 (t, 4H) ; 6,01 (s, 2H) ; 6,35 (d, 1H) ; 6,89 (m, 2H) ; 7,01 (txd, 1H) ; 7,12 (txd, 1H) ; 7,19 (q,1H) ; 7,34 (s, 1H) ;7,52 (m, 2H) ; 7,82 (dxd, 1H) ; 8,31 (s, 1H) ; 10,28 (s, 1H).

**Exemple 4 (Composé N˚7)**

*N*-[6-(3-hydroxypyrrolidin-1-yl)-pyridin-3-yl]-5-fluoro-1-(3-fluorobenzyl)-1*H*-indole-2-carboxamide

On chauffe durant 20 minutes dans un four microonde réglé à 200˚C sous 300 watt, un mélange de 0,4 g (1,01 mmole) de *N*-[6-chloropyridin-3-yl]-5-fluoro-1-(3-fluorobenzyl)-1*H*-indole-2-carboxamide préparé à l'étape 3.1 et 0,81 mL (10,06 mmoles) de 3-hydroxypyrrolidine dans 1,2 mL de N-méthylpyrrolidinone. Le mélange réactionnel est ensuite versé dans 50 mL d'eau. On recueille un solide par filtration que l'on purifie par chromatographie sur colonne de silice en éluant avec un mélange de dichlorométhane et de méthanol. Le solide résultant est ensuite recristallisé dans un mélange de méthanol et de dichlorométhane. On recueille par filtration 0,22 g d'un solide séché sous pression réduite.
Point de fusion : 229 - 230 ˚C
R.M.N. $^1H$ (DMSO $D_6$), δ (ppm): 1,9 (m, 1H) ; 2,04 (m, 1H) ; 3,3-3,47 (m, 4H) ; 4,49 (s, 1H) ; 4,92 (s, 1H) ; 5,9 (s, 2H) ; 6,47 (d, 1H) ; 6,91 (m, 2H) ; 7,05 (txd, 1H) ; 7,12 (txd, 1H) ; 7,31 (q,1H) ; 7,39 (s, 1H) ;7,55 (m, 2H) ; 7,82 (dxd, 1H) ; 8,37 (s, 1H) ; 10,22 (s, 1H).

**Exemple 5 (Composé N˚8)**

*N*-[5-méthoxy-6-(pyrrolidin-1-yl)-pyridin-3-yl]-5-fluoro-1-(3-fluorobenzyl)-1*H*-indole-2-carboxamide

5.1 3-méthoxy-5-nitro-2-(pyrrolidin-1-yl)pyridine

On chauffe à 100˚C durant 4 heures une solution de 3 g (15,91 mmoles) de 2-chloro-3-méthoxy-5-nitropyridine et 2 mL (23,86 mmoles) de pyrrolidine dans 30 mL de diméthylformamide. Le mélange réactionnel est ensuite concentré sous pression réduite puis repris dans 100 mL d'eau et extrait trois fois avec 100 mL d'acétate d'éthyle. Les phases organiques sont réunies puis lavées deux fois par 50 mL d'eau puis une fois avec 50 ml d'une solution saturée en chlorure de sodium, séchées sur sulfate de sodium puis concentrées sous pression réduite. On isole ainsi 3,47 g du composé attendu sous la forme d'une poudre orangée.
R.M.N. $^1H$ (DMSO $D_6$), δ (ppm): 1,9 (m, 4H) ; 3,79 (m, 4H) ; 3,88 (s, 3H) ; 7,62 (s, 1H) ; 8,69 (s, 1H).

5.2 3-méthoxy-2-(pyrrolidin-1-yl)-4-aminopyridine (composé Va)

Une suspension de 3,4 g (15,54 mmoles) de 3-méthoxy-5-nitro-2-(pyrrolidin-1-yl)-pyridine, obtenu à l'étape 5.1, et de 0,33 g de palladium sur charbon 10% dans 40 mL d'éthanol est agitée 5 heures à 20°C sous 5,6 bars d'hydrogène. Le mélange réactionnel est ensuite filtré sur un tampon de célite, concentré sous pression réduite puis purifié par chromatographie sur colonne de silice en éluant avec un mélange de dichlorométhane et de méthanol. On isole ainsi 0,9 g du composé attendu qui sera utilisé tel quel dans la suite de la synthèse.
R.M.N. $^1$H ($CDCl_3$), δ (ppm): 2,5 (m, 4H) ; 3,23 (pic élargi, 2H) ; 3,49 (m, 4H) ; 3,91 (s, 3H) ; 5,90 (s, 1H) ; 7,71 (s, 1H).

5.3 *N*-[5-méthoxy-6-(pyrrolidin-1-yl)-pyridin-3-yl]-5-fluoro-1-(3-fluorobenzyl)-1*H*-indole-2-carboxamide (Composé n° 8)

On agite durant 15 minutes à 20°C une solution de 0,5 g (1,74 mmole) d'acide 5-fluoro-1-(3-fluorobenzyl)-1*H*-indole-2-carboxylique, préparé à l'étape 1.1, de 0,33 g (1,74 mmole) de *N*-(3-diméthylaminopropyl)-*N'*-éthylcarbodiimide et de 0,23 g (1,74 mmole) de *N*-1-hydroxybenzotriazole dans 10 mL de diméthylformamide. On ajoute ensuite au milieu réactionnel 0,43 g (2,26 mmoles) du composé (Va) préparé à l'étape 5.2. Le mélange réactionnel est ensuite agité 12 heures à 20°C puis concentré sous pression réduite, versé sur 100 mL d'eau et extrait deux fois avec 100 mL d'acétate d'éthyle. Les phases organiques sont réunies puis lavées deux fois par 50 mL d'eau puis une fois avec 50 ml d'une solution saturée en chlorure de sodium, séchées sur sulfate de sodium puis concentrées sous pression réduite. Le produit obtenu est purifié par chromatographie sur colonne de silice en éluant avec un mélange de dichlorométhane et de méthanol. On isole ainsi 0,3 g du composé attendu.
Point de fusion : 172 - 174 °C
R.M.N. $^1$H (DMSO $D_6$), δ (ppm): 1,85 (m, 4H) ; 3,61 (m, 4H) ; 3,78 (s, 3H) ; 5,91 (s, 2H) ;
6,91 (m, 2H) ; 7,07 (txd, 1H) ; 7,15 (txd, 1H) ;7,31 (q, 1H) ; 7,4 (s, 1H) ; 7,58 (m, 3H) ; 8,08 (s, 1H) ; 10,3 (s, 1H).

**<u>Exemple 6</u> (Composé N°16)**

*N*-[4-méthoxy-6-(pyrrolidin-1-yl)-pyridin-3-yl]-5-fluoro-1-(3-fluorobenzyl)-1*H*-indole-2-carboxamide

6.1 4-méthoxy-5-nitro-2-(pyrrolidin-1-yl)-pyridine

On procède suivant la méthode décrite à l'étape 5.1 à partir de 5,4 g (28,64 mmoles) de 2-chloro-4-méthoxy-5-nitropyridine (WO03/080610) et de 4,53 g (63 mmoles) de pyrrolidine. Le produit obtenu est, dans ce cas, purifié par chromatographie sur colonne de silice en éluant avec un mélange d'heptane et d'acétate d'éthyle. On isole ainsi 3 g du produit attendu.
R.M.N. $^1$H (DMSO $D_6$), δ (ppm): 2,17 (m, 4H) ; 3,65 (m, 4H) ; 4,07 (s, 3H) ; 5,79 (s, 1H) ; 8,98 (s, 1H).

6.2 4-méthoxy-2-(pyrrolidin-1-yl)-5-aminopyridine (Composé Vb)

On procède suivant la méthode décrite à l'étape 5.2 à partir de 1,5 g (6,72 mmoles) de 4-méthoxy-5-nitro-2-(pyrrolidin-1-yl)-pyridine, obtenu à l'étape 6.1, et de 0,15 g de palladium sur charbon à 10%. On obtient ainsi 1,25 g du produit attendu.
R.M.N. $^1$H (DMSO $D_6$), δ (ppm): 1,81 (m, 4H) ; 3,29 (m, 4H) ; 3,71 (s, 3H) ; 4,52 (pic élargi, 2H) ; 6,61 (s, 1H) ; 7,16 (s, 1H).

6.3 *N*-[4-méthoxy-6-(pyrrolidin-1-yl)-pyridin-3-yl]-5-fluoro-1-(3-fluorobenzyl)-1H-indole-2-carboxamide (Composé n° 16)

On procède suivant la méthode décrite dans l'exemple 2 à partir de 0,5 g (1,74 mmole) d'acide 5-fluoro-1-(3-fluorobenzyl)-1*H*-indole-2-carboxylique (exemple 1.1) et de 0,424 g (2,09 mmoles) de 4-méthoxy-2-(pyrrolidin-1-yl)-5-aminopyridine, obtenue à l'étape 6.1 (Composé Vb). On isole ainsi 0,59 g du produit attendu.
Point de fusion : 196 - 198 °C
R.M.N. $^1$H ($CDCl_3$), δ (ppm): 2,01 (m, 4H) ; 3,49 (m, 4H) ; 3,91 (s, 3H) ; 5,86 (m, 3H) ; 6,79 (dxd 1H) ; 6,9 (m, 2H) ;7,02 (m, 2H) ; 7,23 (m, 2H) ;7,34 (dxd, 1H) ; 7,9 (m, 1H) ; 8,85 (s, 1H).

**<u>Exemple 7</u> (Composé N°20)**

*N*-[6-(1-oxy-pyrrolidin-1-yl)-pyridin-3-yl]-5-fluoro-1-(3-fluorobenzyl)-1*H*-indole-2-carboxamide

On agite pendant 24 heures à 20°C une solution de 0,5 g (1,16 mmole) de *N*-[6-(pyrrolidin-1-yl)-pyridin-3-yl]-5-fluoro-1-(3-fluorobenzyl)-1H-indole-2-carboxamide (composé n°2 préparé selon la méthode décrite dans l'exemple

2) et de 0,31 g (1,27 mmole) d'acide métachloroperbenzoïque (70%) dans 20 mL de dichlorométhane. On ajoute 100 ml de dichlorométhane au mélange réactionnel qui est ensuite lavé successivement avec 20 mL d'une solution saturée en carbonate de sodium puis 3 fois avec 20 mL d'eau, séché sur sulfate de magnésium puis concentré sous pression réduite. Le produit obtenu est ensuite purifié par chromatographie sur colonne de silice en éluant avec un mélange de dichlorométhane et de méthanol. On isole ainsi 0,23 g du produit attendu.
Point de fusion : 140 - 143 °C
R.M.N. $^1H$ (DMSO $D_6$), δ (ppm): 2,1 (m, 2H) ; 2,35 (m, 2H) ; 3,31 (m, 2H) ; 4,08 (m, 2H) ; 5,91 (s, 2H) ; 6,9 (m, 2H) ; 7,04 (txd, 1H) ; 7,2 (txd, 1H) ; 7,32 (m, 1H) ; 7,51 (s, 1H) ; 7,62 (m, 2H) ; 8,41 (m, 2H) ; 8,8 (s, 1H) ; 10,91 (s, 1H)

**Exemple 8 (Composé N°21)**

*N*-[2-(pyrrolidin-1-yl)-pyrimidin-5-yl]-5-fluoro-1-(3-fluorobenzyl)-1*H*-indole-2-carboxamide

On procède suivant la méthode décrite dans l'exemple 2 à partir d'acide 5-fluoro-1-(3-fluorobenzyl)-1*H*-indole-2-carboxylique (exemple 1.1) et de 2-(pyrrolidin-1-yl)-5-aminopyrimidine (US20060281772).
Point de fusion : 220 - 222 °C
R.M.N. $^1H$ (DMSO $D_6$), δ (ppm): 1,97 (m, 4H) ; 3,51 (m, 4H) ; 5,89 (s, 2H) ; 6,91 (m, 2H) ; 7,05 (txd, 1H) ;7,16 (txd, 1H) ; 7,31 (m, 1H) ;7,40 (s, 1H) ; 7,57 (m, 2H) ; 8,61 (s, 2H) ; 10, 32 (s, 1H).

**Exemple 9 (Composé N°27)**

*N*-[2-méthyl-6-(pyrrolidin-1-yl)-pyridin-3-yl]-5-fluoro-1-(3-fluorobenzyl)-1*H*-indole-2-carboxamide

9.1 2-méthyl-6-(pyrrolidin-1-yl)-3-nitropyridine

On chauffe 2 heures à 110°C une suspension de 0,5 g (2,9 mmoles) de 6-chloro-3-nitro-2-picoline, de 0,73 mL (8,69 mmoles) de pyrrolidine et de 0,8 g (5,79 mmoles) de carbonate de potassium. Le mélange réactionnel est ensuite concentré sous pression réduite puis versé sur 100 mL d'eau. On recueille par filtration un solide que l'on sèche pour obtenir 0,55 g du produit attendu sous la forme d'un solide jaune qui sera utilisé tel quel dans la suite de la synthèse.

9.2 2-méthyl-6-(pyrrolidin-1-yl)-3-aminopyridine (composé n° Vd)

On procède suivant la méthode décrite à l'étape 5.2 à partir de 0,55 g (2,65 mmoles) de 2-méthyl-6-(pyrrolidin-1-yl)-3-nitropyridine, préparé à l'étape 9.1, et de 0,1 g de palladium sur charbon à 10%. On obtient ainsi 0,34 g du produit attendu qui sera utilisé tel quel dans la suite de la synthèse.
R.M.N. $^1H$ ($CDCl_3$), δ (ppm): 1.89 (m, 4H) ; 2,23 (s, 3H) ; 2,98 (pic élargi, 2H) ; 3,31 (m, 4H) ; 6,03 (d, 1H) ; 6,82 (d, 1H).

9.3 *N*-[2-méthyl-6-(pyrrolidin-1-yl)-pyridin-3-yl]-5-fluoro-1-(3-fluorobenzyl)-1*H*-indole-2-carboxamide (Composé n° 27)

On procède suivant la méthode décrite dans l'exemple 5.3 à partir de 0,4 g (1,39 mmole) d'acide 5-fluoro-1-(3-fluorobenzyl)-1*H*-indole-2-carboxylique (exemple 1.1) et de 0,32 g (1,81 mmole) de 2-méthyl-6-(pyrrolidin-1-yl)-3-aminopyridine (composé n° Vd), décrit à l'étape 9.2. On isole ainsi 0,3 g du produit attendu.
Point de fusion : 213 - 214 °C
R.M.N. $^1H$ (DMSO $D_6$), δ (ppm): 1,92 (m, 4H) ; 2,21 (s, 3H) ; 3,49 (m, 4H) ; 5,88 (s, 2H) ; 6,29 (d, 1H) ; 6,87 (d, 1H) ; 6,92 (d, 1H) ; 7,05 (txd, 1H) ; 7,15 (txd, 1H) ; 7,38 (m, 2H) ; 7,39 (s, 1H) ; 7,52 (d, 1H) ; 7,67 (m, 1H) ; 9,91 (s, 1H).

**Exemple 10 (Composé N°24)**

*N*-[6-(3-hydroxyazétidin-1-yl)-pyridin-3-yl]-5-fluoro-1-(3-fluorobenzyl)-1*H*-indole-2-carboxamide

10.1 2-(3-hydroxyazétidin-1-yl)-5-nitropyridine

On procède suivant la méthode décrite à l'étape 5.1 à partir de 7 g (42,83 mmoles) de 2-chloro-5-nitropyridine et 5,75 g (51,4 mmoles) de chlorhydrate de 3-azétidinol. Dans ce cas, on ajoute également 13 g (128,45 mmoles) de triéthylamine. On isole ainsi 8 g du produit attendu.
Point de fusion : 175 - 176 °C
R.M.N. $^1H$ (DMSO $D_6$), δ (ppm): 3,85 (m, 2H) ; 4,36 (m, 2H) ; 4,62 (m, 1H) ; 5,88 (d, 1H) ; 6,4 (d, 1H) ; 8,18 (dxd, 1H) ; 8,92 (s, 1H).

10.2 2-(3-hydroxyazétidin-1-yl)-5-aminopyridine (Composé Ve)

On procède suivant la méthode décrite à l'étape 5.2 à partir de 5,5 g (28,18 mmoles) de 2-(3-hydroxyazétidin-1-yl)-5-nitropyridine, préparé à l'étape 10.1, et de 0,5 g de palladium sur charbon à 10%. On obtient ainsi 4,5 g du produit attendu sous la forme de cristaux violets.
Point de fusion : 148 - 150 °C
R.M.N. $^1$H (DMSO $D_6$), δ (ppm): 3,49 (m, 2H) ; 3,98 (m, 2H) ; 4,48 (pic élargi, 3H) ; 5,48 (pic élargi, 1H) ; 6,22 (d, 1H) ; 6,91 (d, 1H) ; 7,58 (s, 1H).

10.3 *N*-[6-(3-hydroxyazétidin-1-yl)-pyridin-3-yl]-5-fluoro-1-(3-fluorobenzyl)-1*H*-indole-2-carboxamide (composé n°24)

On procède suivant la méthode décrite dans l'exemple 2 à partir de 0,5 g (1,74 mmole) d'acide 5-fluoro-1-(3-fluorcbenzyl)-1*H*-indole-2-carboxylique (exemple 1.1) et de 0,35 g (2,09 mmoles) de 2-(3-hydroxyazétidin-1-yl)-5-aminopyridine, obtenu à l'étape 10.2 (Composé Ve).
Point de fusion : 199 - 200 °C
R.M.N. $^1$H (DMSO $D_6$), δ (ppm): 3,67 (m, 2H) ; 4,13 (m, 2H) ; 4,58 (m, 1H) ; 5,61 (m, 1H) ; 5,91 (s, 2H) ; 6,42 (d, 1H) ; 6,91 (m, 2H) ;7,05 (m, 1H) ;7,18 (txd, 1H) ; 7,36 (m, 1H) ;7,42 (s, 1H) ; 7,58 (m, 2H) ; 7,88 (dxd, 1H) ; 8,39 (s, 1H) ; 10,35 (s, 1H).

**Exemple 11 (Composé N°53)**

*N*-[6-(pyrrolidin-1-yl)-4-(trifluorométhyl)pyridin-3-yl]-5-fluoro-1-(3-fluorobenzyl)-1*H*-indole-2-carboxamide

11.1 6-(pyrrolidin-1-yl)-4-(trifluorométhyl)pyridine-3-carboxylate de méthyle

On chauffe à 100°C, pendant 3 heures, un mélange de 2,5 g (10,43 mmoles) de 6-chloro-4-trifluorométhyl-nicotinate de méthyle, de 2,88 g (20,87 mmoles) de carbonate de potassium et de 2,61 mL (31,3 mmoles) de pyrrolidine dans 90 mL de diméthylformamide. Le mélange réactionnel est ensuite concentré sous pression réduite puis repris dans 100 mL d'eau. On recueille, par filtration, un précipité que l'on lave avec 150 mL d'eau. On isole, après séchage sous pression réduite, 2,5 g du composé attendu.
R.M.N. $^1$H ($CDCl_3$), δ (ppm): 2,11 (m, 4H) ; 3,61 (pic élargi, 4H) ; 3,93 (s, 3H) ; 6,69 (s, 1H) ; 8,89 (s, 1H).

11.2 Acide 6-(pyrrolidin-1-yl)-4-(trifluorométhyl)pyridine-3-carboxylique

On agite pendant 24 heures à 20°C un mélange de 2,5 g (9,12 mmoles) de 6-(pyrrolidin-1-yl)-4-(trifluorométhyl) pyridine-3-carboxylate de méthyle, obtenu à l'étape 11.1, et de 0,76 g (13,67 mmoles) de potasse dans 50 mL de méthanol et 2 mL d'eau. Le mélange est ensuite concentré sous pression réduite. On ajoute ensuite 100 mL d'eau et la solution est lavée avec 100 mL de dichlorométhane puis acidifiée à pH 4 par ajouts d'acide chlorhydrique concentré. On recueille un précipité par filtration que l'on lave avec 50 mL d'eau. On isole, après séchage sous pression réduite, 2,2 g du composé attendu.
R.M.N. $^1$H (DMSO $D_6$), δ (ppm): 1,99 (s, 4H) ; 3,51 (pic élargi, 4H) ; 6,71 (s, 1H) ; 8,72 (s, 1H).

11.3 6-(pyrrolidin-1-yl)-4-trifluorométhyl-3-(terbutoxycarbonylamino)pyridine

On chauffe pendant 5 heures à 90°C un mélange de 2,2 g (8,45 mmoles) d'acide 6-(pyrrolidin-1-yl)-4-(trifluorométhyl)pyridine-3-carboxylique, obtenu à l'étape 11.2, de 2,37 mL (10,99 mmoles) de diphénylphosphorylazide et de 2,95 mL (21,14 mmoles) de triéthylamine dans 25 mL de terbutanol. Le mélange réactionnel est ensuite concentré sous pression réduite, repris avec 50 mL d'eau puis extrait 3 fois avec 50 mL de dichlorométhane. Les phases organiques sont réunies, lavées avec 50 ml d'eau, séchées sur sulfate de sodium puis concentrées sous pression réduite. L'huile obtenue est purifiée par chromatographie sur colonne de silice en éluant avec un mélange de dichlorométhane et de méthanol. On isole ainsi 1,05 g du produit attendu.
R.M.N. $^1$H ($CDCl_3$), δ (ppm): 1,53 (s, 9H) ; 2,09 (m, 4H) ; 3,51 (m, 4H) ; 6,2 (pic élargi, 1H) ; 6,52 (s, 1H) ; 8,39 (pic élargi, 1H).

11.4 Chlorhydrate de 6-(pyrrolidin-1-yl)-4-trifluorométhyl-3-aminopyridine (amine Vg)

On agite pendant 5 heures à reflux une solution de 1 g (3,02 mmoles) de 6-(pyrrolidin-1-yl)-4-trifluorométhyl-3-(terbutoxycarbonylamino)pyridine, préparée à l'étape 11.3, dans 11 mL d'acide chlorhydrique 4N dans le dioxane. On

ajoute ensuite 200 mL d'éther éthylique au mélange réactionnel refroidi. On recueille 0,8 g d'un précipité par filtration.
Point de fusion : 207 - 209 °C ;
R.M.N. $^1$H (DMSO $D_6$), δ (ppm): 2 (m, 4H) ; 3,52 (m, 4H) ; 7,1 (s, 1H) ; 7,49 (pic élargi, 2H) ; 7,92 (s, 1H).

11.5 *N*-[6-(pyrrolidin-1-yl)-4-trifluorométhylpyridin-3-yl]-5-fluoro-1-(3-fluorobenzyl)-1*H*-indole-2-carboxamide (composé n° 53)

On procède suivant la méthode décrite à l'étape 5.3, à partir de 0,3 g (1,04 mmole) d'acide 5-fluoro-1-(3-fluorobenzyl)-1*H*-indole-2-carboxylique (exemple 1.1), de 0,363 g (1,36 mmole) et de chlorhydrate de 6-(pyrrolidin-1-yl)-4-trifluorométhyl-3-aminopyridine, préparée à l'étape 11.4, en présence de 0,22 mL (1,57 mmole) de triéthylamine.
Point de fusion: 170 - 171 °C ;
R.M.N. $^1$H (DMSO $D_6$), δ (ppm): 1,99 (m, 4H) ; 3,49 (m, 4H) ; 5,87 (s, 2H) ; 6,7 (s, 1H) ; 6,86 - 7,22 (m, 4H) ; 7,31 (m, 1H) ; 7,4 (s, 1H) ; 7,57 (dxd, 1H) ; 7,65 (m, 1H) ; 8,11 (s, 1H).

**<u>Exemple 12</u> (Composé N°28)**

*N*-[6-(3-aza-bicyclo[3.1.0]hex-3-yl)-pyridin-3-yl]-5-fluoro-1-(3-fluorobenzyl)-1*H*-indole-2-carboxamide

12.1 2-(3-aza-bicyclo[3.1.0]hex-3-yl)-5-nitropyridine

On agite à température ambiante durant 36 heures une suspension de 0,2 g (1,26 mmole) de 2-chloro-5-nitropyridine, de 0,166 g (1,39 mmole) de chlorhydrate de 3-azabicyclo[3.1.0]hexane (Bioorg.&Med.Chem. Lett. 2005, 15(8) 2093), et de 0,52 g (3,78 mmoles) de carbonate de potassium dans 5 mL de diméthylformamide. Le mélange est ensuite versé sur 5 mL d'eau puis extrait 3 fois avec 20 mL d'acétate d'éthyle. Les phases organiques sont ensuite rassemblées, séchées sur sulfate de magnésium puis concentrées sous pression réduite. On obtient ainsi 0,22 g du produit attendu que l'on utilise tel quel dans la suite de la synthèse.

12.2 2-(3-aza-bicyclo[3.1.0]hex-3-yl)-5-aminopyridine (Vf)

On procède suivant la méthode utilisé à l'exemple 5.2 à partir de 0,2 g (0,97 mole) de 2-(3-aza-bicyclo[3.1.0]hex-3-yl)-5-nitropyridine, décrit à l'étape 12.1, et de 0,5 g de palladium sur charbon à 10%. On isole ainsi 0,1 g du produit attendu qui sera utilisé tel quel dans l'étape suivante.
R.M.N. $^1$H (DMSO $D_6$), δ (ppm): 0,2 (m, 1H) ; 0,66 (m, 1H) ; 1,6 (m, 2H) ; 3,11 (m, 2H) ; 3,51 (d, 2H) ; 4,32 (pic élargi, 2H) ; 6,25 (d, 1H) ; 6,99 (d,1H) ; 7,55 (s, 1H).

12.3 *N*-[6-(3-aza-bicyclo[3.1.0]hex-3-yl)-pyridin-3-yl]-5-fluoro-1-(3-fluorobenzyl)-1*H*-indole-2-carboxamide (composé n° 28).

On procède suivant la méthode décrite dans l'exemple 2 à partir de 0,15 g (0,52 mmole) de l'acide 5-fluoro-1-(3-fluorobenzyl)-1*H*-indole-2-carboxylique et de 0,1 g (0,57 mmole) de 2-(3-aza-bicyclo[3.1.0]hex-3-yl)-5-aminopyridine, préparé à l'étape 12.2. On isole ainsi 0,17 g du produit attendu.
Point de fusion : 193 - 195 °C
R.M.N. $^1$H (DMSO $D_6$), δ (ppm): 0,02 (m, 1H) ; 0,51 (m, 1H) ; 1,47 (m, 2H) ; 3,41 (d, 2H) ; 5,67 (s, 2H) ; 6,25 (d, 1H) ; 6,69 (m, 2H) ;6,83 (m, 1H) ; 6,93 (m, 1H) ;7,1 (m, 1H) ; 7,15 (s, 1H) ; 7,31 (dxd, 1H) ; 7,39 (m, 1H) ; 7,6 (m, 1H) ; 8,11 (s, 1H) ; 10,03 (s, 1H).

**<u>Exemple 13</u> (Composé N°29)**

*N*-[6-(3-hydroxyazétidin-1-yl)-pyridin-3-yl]-1-(3-fluorobenzyl)-5-trifluorométhyl-1*H*-indole-2-carboxamide

On procède suivant la méthode décrite dans l'exemple 2 à partir de l'acide 5-trifluorométhyl-1-(3-fluorobenzyl)-1*H*-indole-2-carboxylique (WO2006/072736) et de 2-(3-hydroxyazétidin-1-yl)-5-aminopyridine, préparé à l'étape 10.2 de l'exemple 10 (Composé Ve).
Point de fusion : 194 - 196 °C
R.M.N. $^1$H (DMSO $D_6$), δ (ppm): 3,68 (m, 2H) ; 4,17 (m, 2H) ; 4,59 (m, 1H) ; 5,6 (d, 1H) ; 5,93 (s, 2H) ; 6,42 (d, 1H) ; 6,91 (m, 2H) ;7,09 (txd, 1H) ; 7,32 (m, 1H) ;7,55 (m, 2H) ; 7,79 (d, 1H) ; 7,88 (d, 1H) ; 8,2 (s, 1H) ; 8,38 (s, 1H) ; 10,41 (s, 1H).

**Exemple 14 (Composé N°30)**

*N*-[6-(3-hydroxyazétidin-1-yl)-pyridin-3-yl]-1-(3-fluorobenzyl)-6-trifluorométhyl-1*H*-indole-2-carboxamide

On procède suivant la méthode décrite dans l'exemple 2 à partir de d'acide 6-trifluorométhyl-1-(3-fluorobenzyl)-1*H*-indole-2-carboxylique et de 2-(3-hydroxyazétidin-1-yl)-5-aminopyridine, préparé à l'étape 10.2 de l'exemple 10 (Composé Ve).
Point de fusion : 253 - 255 °C
R.M.N. $^1$H (DMSO $D_6$), δ (ppm): 3,67 (m, 2H) ; 4,17 (m, 2H) ; 4,59 (m, 1H) ; 5,6 (d, 1H) ; 6,0 (s, 2H) ; 6,42 (d, 1H) ; 6,91 (m, 2H) ;7,09 (txd, 1H) ; 7,36 (m, 1H) ;7,47 (d, 1H) ; 7,51 (s, 1H) ; 7,85 (d, 1H) ; 7,99 (d, 1H) ; 8,06 (s, 1H) ; 8,33 (s, 1H) ; 10,41 (s, 1H).

**Exemple 15 (Composé N°23)**

*N*-[6-(pyrrolidin-1-yl)pyridin-3-yl]-1-[(pyridin-4-yl)-méthyl]-5-fluoro-1*H*-indole-2-carboxamide

15.1 Acide 5-fluoro-1-[(pyridin-4-yl)-méthyl]-1*H*-indole-2-carboxylique

On chauffe, 2 heures à reflux, une solution de 2,1 g (7,04 mmoles) de 5-fluoro-1-[(pyridin-4-yl)-méthyl]-1*H*-indole-2-carboxylate d'éthyle (WO2007/010144) et de 1,18 g (21,12 mmoles) de potasse dans 80 mL d'éthanol et 2 mL d'eau. Le mélange réactionnel est ensuite concentré sous pression réduite. On additionne 100 mL d'eau et on amène le pH de la solution à pH 8 par addition d'une solution d'acide chlorhydrique concentrée. On recueille, par filtration, un précipité qui est lavé à l'eau puis séché sous pression réduite. On obtient ainsi 1,5 g du produit attendu.
Point de fusion : 282 - 283 °C

15.2 *N*-[6-chloropyridin-3-yl]-5-fluoro-1-[(pyridin-4-yl)-méthyl]-1*H*-indole-2-carboxamide

On procède suivant la méthode décrite dans l'exemple 2 à partir de 0,7 g (2,59 mmoles) d'acide 5-fluoro-1-[(pyridin-4-yl)-méthyl]-1*H*-indole-2-carboxylique, obtenu à l'étape 15.1, et de 0,4 g (3,11 mmoles) de 2-chloro-5-aminopyridine. On isole 0,41 g de produit attendu.
Point de fusion : 244 - 246 °C

15.3 *N*-[6-(pyrrolidin-1-yl)pyridin-3-yl]-1-[(pyridin-4-yl)-méthyl]-5-fluoro-1*H*-indole-2-carboxamide (composé n°23)

On procède suivant la méthode décrite dans l'exemple 3.2 à partir de 0,4 g (1,05 mmole) de *N*-[6-chloropyridin-3-yl]-5-fluoro-1-[(pyridin-4-yl)-méthyl]-1*H*-indole-2-carboxamide, obtenu à l'étape 15.2, et de 2,63 mL (31,51 mmoles) de pyrrolidine. On isole 0,24 g du produit attendu.
Point de fusion : 255 - 257 °C
R.M.N. $^1$H (DMSO $D_6$), δ (ppm): 1,92 (m, 4H) ; 3,38 (m, 2H) ; 5,92 (s, 4H) ; 6,45 (d, 1H) ; 7,0 (d, 2H) ; 7,16 (txd, 1H) ; 7,43 (s, 1H) ;7,57 (m, 2H) ; 7,81 (m, 1H) ;8,31 (s, 1H) ; 8,48 (d, 2H) ; 10,22 (s, 1H).

**Exemple 16 (Composé N°14)**

*N*-[6-(pyrrolidin-1-yl)pyridin-3-yl]-1-[(pyridin-4-yl)-méthyl]-5-trifluorométhyl-1*H*-indole-2-carboxamide

16.1 Acide 5-trifluorométhyl-1-[(pyridin-4-yl)-méthyl]-1*H*-indole-2-carboxylique

On procède suivant la méthode décrite à l'exemple 15.1 à partir de 5-trifluorométhyl-1-[(pyridin-4-yl)-méthyl]-1*H*-indole-2-carboxylate d'éthyle (WO07/010144). Le produit obtenu est utilisé tel quel dans la suite de la synthèse.

16.2 *N*-[6-chloropyridin-3-yl]-1-[(pyridin-4-yl)-méthyl]-5-trifluorométhyl-1*H*-indole-2-carboxamide

On procède suivant la méthode décrite à l'exemple 3.1 à partir de 0,5 g (1,56 mmole) d'acide 5-trifluorométhyl-1-[(pyridin-4-yl)-méthyl]-1*H*-indole-2-carboxylique, préparé à l'étape 16.1, et de 0,22 g (1,72 mmole) de 2-chloro-5-aminopyridine. On isole par cette méthode 0,5 g du produit attendu qui est utilisé tel quel dans la suite de la synthèse.

16.3 *N*-[6-(pyrrolidin-1-yl)pyridin-3-yl]-1-[(pyridin-4-yl)-méthyl]-5-trifluorométhyl-1*H*-indole-2-carboxamide (composé n° 14)

On procède suivant la méthode décrite à l'exemple 4 à partir de 0,5 g (1,16 mmole) de *N*-[6-chloropyridin-3-yl]-1-[(pyridin-4-yl)-méthyl]-5-trifluorométhyl-1H-indole-2-carboxamide, obtenu à l'étape 16.2, et de 2,91 mL (34,82 mmoles) de pyrrolidine. Le mélange est placé dans un tube à pression que l'on chauffe 50 minutes à 170 °C sous 200 watts, au moyen d'un four microonde. Le mélange est ensuite concentré sous pression réduite, repris par 100 mL d'acétate d'éthyle. La phase organique est ensuite lavée deux fois avec 30 mL d'eau puis une fois avec une solution saturée en chlorure de sodium, séchée sur sulfate de magnésium puis concentrée sous pression réduite. Le produit obtenu est purifié par chromatographie sur colonne de silice en éluant avec un mélange de dichlorométhane et de méthanol. On isole ainsi 0,24 g du produit attendu.
Point de fusion : 248 - 250 °C
R.M.N. $^1H$ (DMSO $D_6$), δ (ppm): 1,95 (m, 4H) ; 3,41 (m, 4H) ; 5,98 (s, 2H) ; 6,45 (d, 1H) ; 7,03 (d, 2H) ; 7,59 (m, 2H) ; 7,73 (d, 1H) ;7,81 (d, 1H) ; 8,19 (s, 1H) ;8,32 (s, 1H) ; 8,49 (d, 2H).

**<u>Exemple 17</u> (Composé N°22)**

*N*-[6-(3-aza-bicyclo[3.2.0]hept-3-yl)-pyridin-3-yl]-5-fluoro-1-(3-fluorobenzyl)-1H-indole-2-carboxamide

On chauffe durant 90 minutes dans un four microonde réglé à 150°C sous 150 watt, un mélange de 0,2 g (0,5 mmole) de *N*-[6-chloropyridin-3-yl]-5-fluoro-1-(3-fluorobenzyl)-1*H*-indole-2-carboxamide préparé à l'étape 3.1, de 0,0806 g (0,6 mmoles) de 3-azabicyclo[3.2.0]heptane (J.Med.Chem. 1967, 10(4), 621) et de 0,0564 g (1,01 mmole) de potasse dans 0,5 mL de N-méthylpyrrolidinone. Le mélange réactionnel est ensuite versé dans 20 mL d'eau. Le mélange est extrait par trois fois 30 mL d'acétate d'éthyle. Les phases organiques rassemblées sont lavées avec 30 mL d'eau, séchées sur sulfate de sodium puis concentrées sous pression réduite. Le solide résultant est ensuite purifié par chromatographie sur colonne de silice en éluant avec un mélange de n-heptane et d'acétate d'éthyle. On isole ainsi 35 mg du produit attendu.
Point de fusion : 163 - 165 °C
R.M.N. $^1H$ (DMSO $D_6$), δ (ppm): 1,71 (m, 2H) ; 2,23 (m, 2H) ; 3,02 (m, 2H) ; 3,2 (m, 2H) ; 3,63 (d, 2H) ; 5,9 (s, 2H) ; 6,62 (d, 1H) ; 6,91 (m, 2H) ; 7,05 (txd, 1H) ; 7,16 (txd, 1H) ; 7,33 (m,1H) ; 7,4 (s, 1H) ;7,54 (dxd, 1H) ; 7,6 (m, 1H) ; 7,88 (dxd, 1H) ; 8,4 (s, 1H) ; 10,3 (s, 1H).

Le tableau 2 qui suit illustre les structures chimiques et les propriétés physiques de quelques composés de formule générale (I) selon l'invention.
Dans ce tableau :

- la colonne « PF » renseigne les points de fusion des produits en degrés Celsius (°C) ;
- dans la colonne « Sel/base », « - » représente un composé sous forme de base libre, alors que « HCl » représente un composé sous forme de chlorhydrate et le rapport entre parenthèses est le rapport (acide:base).
- « t-Bu » correspond à un groupe tertiobutyle, « iPr » à un groupe isopropyle, « Et » à un groupe éthyle.

Tableau 2

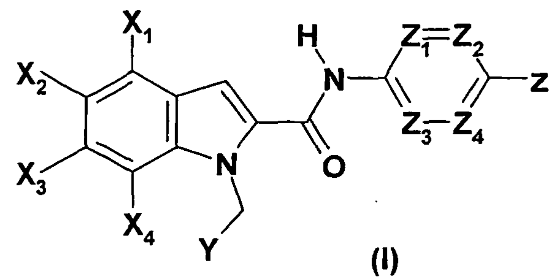

(I)

| N° | $X_1, X_2, X_3, X_4$ | Y | $Z_1, Z_2, Z_3, Z_4$ | Z | Sel / base | PF (°C) |
|---|---|---|---|---|---|---|
| 1 | H, F, H, H | 3-fluorophényle | CH, CH, CH, N | (R)-N-(3-terbutoxy carbonylamino)-pyrrolidinyle | - | 207 - 209 |
| 2 | H, F, H, H | 3-fluorophényle | CH, CH, CH, N | pyrrolidinyle | - | 199 - 201 |
| 3 | H, F, H, H | 3-fluorophényle | CH, CH, CH, N | (*R*)-3-amino-pyrrolidinyle | - | 192 - 194 |
| 4 | H, F, H, H | 3-fluorophényle | CH, CH, CH, N | azétidinyle | - | 227 - 228 |
| 5 | H, $CF_3$, H, H | 3-fluorophényle | CH, CH, CH, N | pyrrolidinyle | - | 185-186 |
| 6 | H, F, H, H | 3-fluorophényle | C-$CH_3$, CH, CH, N | pyrrolidinyle | - | 176 - 178 |
| 7 | H, F, H, H | 3-fluorophényle | CH, CH, CH, N | 3-hydroxy-pyrrolidinyle | - | 229 - 230 |
| 8 | H, F, H, H | 3-fluorophényle | CH, C-$OCH_3$, CH, N | pyrrolidinyle | - | 172 - 174 |
| 9 | H, H, $CF_3$, H | Pyridin-4-yle | CH, CH, CH, N | pyrrolidinyle | - | 261 - 262 |
| 10 | H, F, H, H | Pyridin-4-yle | C-$CH_3$, CH, CH, N | pyrrolidinyle | - | 115 - 120 |
| 11 | H, F, H, H | Pyridin-4-yle | CH, C-$OCH_3$, CH, N | pyrrolidinyle | - | 213 - 215 |
| 12 | H, F, H, H | 3-(pyrrolidin-1yl)phényle | CH, CH, CH, N | pyrrolidinyle | - | 225 - 227 |
| 13 | H, F, H, H | 3-fluorophényle | CH, CH, CH, N | pipéridinyle | - | 190 - 192 |
| 14 | H, $CF_3$, H, H | Pyridin-4-yle | CH, CH, CH, N | pyrrolidinyle | - | 248 - 250 |
| 15 | H, F, H, H | 6-méthylpyridin-2-yle | CH, CH, CH, N | pyrrolidinyle | - | 241 - 243 |

(suite)

| N° | $X_1$, $X_2$, $X_3$, $X_4$ | Y | $Z_1$, $Z_2$, $Z_3$, $Z_4$ | Z | Sel / base | PF (°C) |
|---|---|---|---|---|---|---|
| 16 | H, F, H, H | 3-fluorophényle | C-$OCH_3$, CH, CH, N | pyrrolidinyle | - | 196 - 198 |
| 17 | H, F, H, H | 3-fluorophényle | CH, CH, CH, N | morpholinyle | HCl (1:1) | 246 - 250 |
| 18 | H, F, H, H | Pyridin-4-yle | C-$OCH_3$, CH, CH, N | pyrrolidinyle | - | 225 - 226 |
| 19 | H, F, H, H | 2-méthylpyridin-4-yle | CH, CH, CH, N | pyrrolidinyle | - | 213 - 215 |
| 20 | H, F, H, H | 3-fluorophényle | CH, CH, CH, N | 1-oxy-pyrrolidinyle | - | 140 - 143 |
| 21 | H, F, H, H | 3-fluorophényle | CH, N, CH, N | pyrrolidinyle | - | 220 - 222 |
| 22 | H, F, H, H | 3-fluorophényle | CH, CH, CH, N | 3-azabicyclo [3.2.0]heptyle | - | 163 - 165 |
| 23 | H, F, H, H | Pyridin-4-yle | CH, CH, CH, N | pyrrolidinyle | - | 255 - 257 |
| 24 | H, F, H, H | 3-fluorophényle | CH, CH, CH, N | 3-hydroxy-azétidinyle | - | 199 - 200 |
| 25 | H, $CF_3$, H, H | 3-fluorophényle | CH, CH, CH, N | azétidinyle | - | 222 - 224 |
| 26 | H, F, H, H | Pyridin-4-yle | CH, CH, CH, N | azétidinyle | - | 212 - 214 |
| 27 | H, F, H, H | 3-fluorophényle | CH, CH, C-$CH_3$, N | pyrrolidinyle | - | 213-214 |
| 28 | H, F, H, H | 3-fluorophényle | CH, CH, CH, N | 3-azabicyclo [3.1.0]hexyle | - | 193 - 195 |
| 29 | H, $CF_3$, H, H | 3-fluorophényle | CH, CH, CH, N | 3-hydroxy-azétidinyle | - | 194 - 196 |
| 30 | H, H, $CF_3$, H | 3-fluorophényle | CH, CH, CH, N | 3-hydroxy-azétidinyle | - | 253 - 255 |
| 31 | H,F,H,H | phényle | CH, CH, CH, N | azétidinyle | - | 208 - 210 |
| 32 | $CH_3$, H, $CH_3$, H | 3-fluorophényle | CH, CH, CH, N | azétidinyle | - | 234 - 236 |
| 33 | H, H, t-Bu, H | 3-fluorophényle | CH, CH, CH, N | azétidinyle | - | 204 - 206 |
| 34 | H, H, O-iPr, H | 3-fluorophényle | CH, CH, CH, N | azétidinyle | - | 198 - 200 |
| 35 | H, $SO_2CH_3$, H, H | 3-fluorophényle | CH, CH, CH, N | azétidinyle | - | 258 - 260 |
| 36 | H, H, $N(CH_3)_2$, H | 3-fluorophényle | CH, CH, CH, N | azétidinyle | - | 256 - 258 |
| 37 | H, H, $SCH_3$, H | 3-fluorophényle | CH, CH, CH, N | azétidinyle | - | 206 - 208 |
| 38 | F,H,H,H | 3-fluorophényle | CH, CH, CH, N | azétidinyle | - | 214 - 216 |
| 39 | H, H, H, F | 3-fluorophényle | CH, CH, CH, N | azétidinyle | - | 244 - 246 |
| 40 | H, H, F, H | 3-fluorophényle | CH, CH, CH, N | azétidinyle | - | 210-212 |

(suite)

| N° | $X_1$, $X_2$, $X_3$, $X_4$ | Y | $Z_1$, $Z_2$, $Z_3$, $Z_4$ | Z | Sel / base | PF (°C) |
|---|---|---|---|---|---|---|
| 41 | Cl, H, H, H | 3-fluorophényle | CH, CH, CH, N | azétidinyle | - | 226 - 228 |
| 42 | H, H, Cl, H | 3-fluorophényle | CH, CH, CH, N | azétidinyle | - | 218 - 220 |
| 43 | H, Cl, H, H | 3-fluorophényle | CH, CH, CH, N | azétidinyle | - | 226 - 228 |
| 44 | H, H, H, H | 3-fluorophényle | CH, CH, CH, N | azétidinyle | - | 328 - 330 |
| 45 | $CH_3$, H, H, H | 3-fluorophényle | CH, CH, CH, N | azétidinyle | - | 246 - 248 |
| 46 | H, H, $CH_3$, H | 3-fluorophényle | CH, CH, CH, N | azétidinyle | - | 228 - 230 |
| 47 | H, $CH_3$, H, H | 3-fluorophényle | CH, CH, CH, N | azétidinyle | - | 236 - 238 |
| 48 | H, iPr, H, H | 3-fluorophényle | CH, CH, CH, N | azétidinyle | - | 204 - 206 |
| 49 | H, t-Bu, H, H | 3-fluorophényle | CH, CH, CH, N | azétidinyle | - | 212 - 214 |
| 50 | H, H, Et, H | 3-fluorophényle | CH, CH, CH, N | azétidinyle | - | 196 - 198 |
| 51 | H, H, iPr, H | 3-fluorophényle | CH, CH, CH, N | azétidinyle | - | 184 - 186 |
| 52 | H, H, $CF_3$, H | 3-fluorophényle | CH, CH, CH, N | azétidinyle | - | 220 - 222 |
| 53 | H, F, H, H | 3-fluorophényle | C-$CF_3$, CH, CH, N | pyrrolidinyle | - | 170 - 171 |

Les données RMN de certains composés du tableau sont décrites à la suite, à titre d'exemples.

Composé n° 25 :
R.M.N. $^1H$ (DMSO $D_6$), δ (ppm): 2,31 (m, 2H) ; 3,92 (t, 4H) ; 5,92 (s, 2H) ; 6,4 (d, 1H) ; 6,94 (m, 2H) ; 7,09 (txd, 1H) ; 7,37 (m, 1H) ;7,57 (m, 2H) ; 7,79 (d, 1H) ; 7,87 (dxd, 1H) ; 8,2 (s, 1H) ; 8,36 (d, 2H) ; 10,41 (s, 1H).

Composé n° 26 :
R.M.N. $^1H$ (DMSO $D_6$), δ (ppm): 2,32 (m, 2H) ; 3,92 (t, 4H) ; 5,91 (s, 2H) ; 6,38 (d, 1H) ; 6,99 (d, 2H) ; 7,15 (txd, 1H) ; 7,41 (s, 1H) ;7,57 (m, 2H) ; 7,81 (m, 1H) ; 8,43 (m, 1H) ; 8,46 (d, 2H) ; 10,4 (s, 1H).

Composé n° 35 :
R.M.N. $^1H$ (DMSO $D_6$), δ (ppm): 2,32 (m, 2H) ; 3,21 (s, 3H) ; 3,92 (t, 4H) ; 5,98 (s, 2H) ; 6,4 (d, 1H) ; 6,92 (d, 2H) ; 7,09 (txd, 1H) ; 7,35 (m, 1H) ;7,6 (s, 1H) ; 7,81 (m, 3H) ; 8,39 (m, 2H) ; 10,42 (s, 1H).

Composé n° 38 :
R.M.N. $^1H$ (DMSO $D_6$), δ (ppm): 2,31 (m, 2H) ; 3,92 (t, 4H) ; 5,92 (s, 2H) ; 6,4 (d, 1H) ; 6,93 (m, 3H) ; 7,08 (txd, 1H) ; 7,31 (m, 2H) ;7,41 (m, 1H) ; 7,61 (s, 1H) ; 7,86 (dxd, 1H) ; 8,38 (s, 1H) ; 10,3 (s, 1H).

Composé n° 40 :
R.M.N. $^1H$ (DMSO $D_6$), δ (ppm): 2,31 (m, 2H) ; 3,90 (t, 4H) ; 5,89 (s, 2H) ; 6,4 (d, 1H) ; 6,91 (m, 2H) ; 7,06 (m, 2H) ; 7,33 (m, 1H) ;7,41 (s, 1H) ; 7,47 (dxd, 1H) ; 7,78 (m, 1H) ; 7,86 (m, 1H) ; 8,35 (s, 1H) ; 10,23 (s, 1H).

Composé n° 45 :
R.M.N. $^1H$ (DMSO $D_6$), δ (ppm): 2,31 (m, 2H) ; 2,56 (s, 3H) ; 3,91 (t, 4H) ; 5,9 (s, 2H) ; 6,4 (d, 1H) ; 6,91 (m, 3H) ; 7,04 (txd, 1H) ; 7,18 (m, 1H) ;7,31 (m, 2H) ; 7,49 (s, 1H) ; 7,88 (m, 1H) ; 8,38 (s, 1H) ; 10,25 (s, 1H).

Composé n° 46 :
R.M.N. $^1H$ (DMSO $D_6$), δ (ppm): 2,31 (m, 2H) ; 2,41 (s, 3H) ; 3,91 (t, 4H) ; 5,85 (s, 2H) ; 6,4 (d, 1H) ; 6,82-7,09 (m, 4H) ; 7,35 (m, 3H) ; 7,61 (d, 1H) ; 7,83 (dxd, 1H) ; 8,36 (s, 1H) ; 10,21 (s, 1H).

Composé n° 47 :
R.M.N. $^1H$ (DMSO $D_6$), δ (ppm): 2,29 (m, 2H) ; 2,4 (s, 3H) ; 3,91 (t, 4H) ; 5,89 (s, 2H) ; 6,39 (d, 1H) ; 6,82-6,91 (m, 2H) ; 7,02 (txd, 1H) ; 7,12 (dxd, 1H) ; 7,31 (m, 2H) ; 7,41 (d, 1H) ; 7,51 (s, 1H) ; 7,87 (dxd, 1H) ; 8,34 (s, 1H) ; 10,23 (s, 1H).

Composé n° 49 :
R.M.N. $^1H$ (DMSO $D_6$), δ (ppm): 1,32 (s, 9H) ; 2,3 (m, 2H) ; 3,91 (t, 4H) ; 5,85 (s, 2H) ; 6,38 (d, 1H) ; 6,92 (m, 2H) ; 7,03 (txd, 1H) ; 7,35 (m, 4H) ;7,66 (s, 1H) ; 7,85 (dxd, 1H) ; 8,43 (s, 1H) ; 10,19 (s, 1H).

Composé n° 52 :
R.M.N. $^1H$ (DMSO $D_6$), δ (ppm): 2,31 (m, 2H) ; 3,91 (t, 4H) ; 6 (s, 2H) ; 6,39 (d, 1H) ; 6,91 (m, 2H) ; 7,07 (txd, 1H) ; 7,32 (m, 1H) ;7,45 (d, 1H) ; 7,49 (s, 1H) ; 7,82 (dxd, 1H) ; 7,92 (d, 1H) ; 8,02 (s, 1H) ; 8,34 (s, 1H) ; 10,41 (s, 1H).

Les composés de l'invention ont été soumis à des essais pharmacologiques *in vitro* et *in vivo* qui ont mis en évidence leur intérêt comme substances à activités thérapeutiques.
Les composés de l'invention présentent également des caractéristiques de solubilité dans l'eau qui favorise une bonne activité *in vivo.*

Test d'inhibition du courant induit par la capsaïcine sur les DRG de rat

- Culture primaire de cellules de ganglions de racine dorsale (DRG) de rat :
  Les neurones du DRG expriment naturellement le récepteur TRPV1.
  Les cultures primaires de DRG de rats nouveaux nés sont préparées à partir de ratons de un jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 μg/ml gentamicine et 50 ng/ml de NGF, puis déposées sur des lamelles de verre

recouvertes de laminine (0.25 x 106 cellules par lamelle) qui sont ensuite placées dans des boîtes 12 puits Corning. Les cellules sont incubées à 37°C en atmosphère humidifiée contenant 5% de $CO_2$ et 95% d'air. De la cytosine β-D-arabinoside (1 μM) est ajoutée 48 h après la mise en culture, pour prévenir le développement des cellules non neuronales. Les lamelles sont transférées dans les chambres expérimentales pour les études de patch-clamp après 7-10 jours de culture.

- Electrophysiologie :
Les chambres de mesure (volume 800 μl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 ml/min) à l'aide d'un distributeur de solutions acceptant 8 entrées et dont la sortie unique, constituée par un tube de polyéthylène (ouverture 500μm) est placée à moins de 3 mm de la cellule étudiée. La configuration "cellule entière" de la technique de patch-clamp a été utilisée. Les pipettes en verre borosilicaté (résistance 5-10 MOhms) sont approchées de la cellule grâce à un micromanipulateur piézoélectrique 3D (Burleigh, PC1000). Les courants globaux (potentiel de membrane fixé à -60 mV) sont enregistrés avec un amplificateur Axopatch 1D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.

L'application d'une solution micromolaire de capsaïcine provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à une concentration donnée (concentration de 10 nM ou de 0,1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.

Les pourcentages d'inhibition de la réponse capsaïcine (1 microM) sont compris entre 20% et 100% pour les composés les plus actifs de l'invention testés à la concentration de 10 nM à 0,1 nM (voir exemple dans le tableau 3). Les composés de l'invention sont donc des antagonistes efficaces in vitro des récepteurs de type TRPV1.

Tableau 3

| N°composé | % inhibition en patch DRG |
|---|---|
| 2 | 46% (1 nM) |
| 4 | 89% (1 nM) |
| 7 | 85% (10 nM) |
| 21 | 79% (10 nM) |

Test d'irritation cornéenne souris

Le caractère irritant de la capsaïcine est aisément apprécié au niveau de la cornée puisque cet organe est un des plus innervé par les fibres C. Dans ce contexte, d'après des expériences préliminaires, l'application d'une très faible quantité de capsaïcine (2 μl à une concentration de 160 μM) à la surface de la cornée d'un animal entraîne un certain nombre de comportements stéréotypés liés à l'irritation et qu'il est facile de répertorier. Parmi ceux-ci, on note : clignement de l'oeil, frottement de l'oeil instillé par la patte avant ipsilatérale, frottement de la face avec les deux pattes avant, grattement de la face ipsilatérale par la patte arrière. La durée de ces comportements ne dépasse pas les 2 minutes d'observation, et l'animal reprend alors son activité normale. Son aspect est par ailleurs également normal. La souris n'est pas recluse dans un coin avec les poils hérissés et ne développe aucun signe observable de souffrance. On peut en conclure que la durée d'action de la capsaïcine à ces doses est inférieure à 2 minutes.

Résumé de la méthodologie :

Le principe de ia série d'expériences est de déterminer si les composés de l'invention peuvent influencer la réponse comportementale induite par une quantité donnée de capsaïcine. La capsaïcine est initialement diluée à 25 mM dans le DMSO et diluée, pour son utilisation finale, dans le sérum physiologique avec du Tween 80 à 10%. Il apparaît, à partir d'études contrôles que dans ces conditions, le solvant n'a aucun effet.
En pratique, le produit à tester, préparé à 25 mM dans le DMSO et dilué pour son utilisation finale dans le sérum physiologique avec 10% de Tween 80 à la plus forte concentration de 500 μM, est administré en application locale à la surface de la cornée sous un volume de 2 μl, 10 minutes avant l'application de la capsaicine. L'animal reçoit l'instillation

oculaire de 2 µl d'une solution de capsaïcine à 160 µM préparée comme indiqué ci-dessus. Au cours d'une observation de 2 minutes suivant l'instillation, le nombre de frottements de l'oeil instillé par la patte antérieure ipsilatérale est compté pour chaque animal.
Pour un groupe donné, le pourcentage de protection est calculé comme suit :

P= 100 – ((nombre moyen de grattages du groupe traité par le composé / nombre moyen de grattages du groupe traité par le solvant) x 100)

Ce pourcentage de protection est moyenné pour chaque groupe d'animaux (n = nombre d'animaux testés avec le composé de l'invention).
Les pourcentages de protection évalués, dans ce modèle, pour les composés de l'invention les plus actifs, utilisés à la concentration de 500µM, sont compris entre 20% et 100% (voir exemple dans le tableau 4) :

**Tableau 4**

| n'composé | % P 500µM |
|---|---|
| 2 | 72% |

Les résultats de ces essais montrent que les composés les plus actifs de l'invention bloquent les effets induits par la stimulation des récepteurs TRPV1.
Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.
Ainsi, selon un autre de ses aspects, l'invention a pour objet des médicaments qui comprennent un composé de formule (I), ou un sel pharmaceutiquement acceptable, ou encore un hydrate ou un solvat dudit composé.
Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.
Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.
Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.
On peut également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.
Les composés de l'invention peuvent également être utilisés pour la préparation d'un médicament destiné à traiter le diabète.
De même, les produits de la présente invention peuvent être utiles dans la prévention et/ou le traitement des désordres respiratoires tels que l'asthme, la toux, la COPD, la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.
Les composés de l'invention peuvent également être utilisés pour la préparation d'un médicament destiné à traiter la dépression.
Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du

métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I)

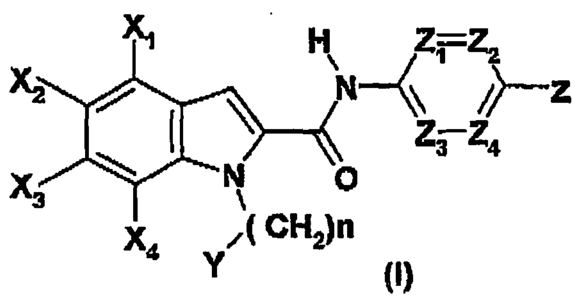

(I)

dans laquelle

$X_1$ représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, cyano, $C(O)NR_1R_2$, nitro, $C_1$-$C_6$-thioalkyle, -S(O)-$C_1$-$C_6$-alkyle, -$S(O)_2$-$C_1$-$C_6$-alkyle, $SO_2NR_1R_2$, aryle-$C_1$-$C_6$-alkylène, aryle ou hétéroaryle, l'aryle et l'hétéroaryle étant éventuellement substitués par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$X_2$ représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène,$C_1$-$C_6$-fluoroalkyle,$C_1$-$C_6$-alcoxyle,$C_3$-$C_7$-cycloalcoxyle,$C_3$-$C_7$-cycloalkyle-$C_1$-$C_6$-alkylène-O-, $C_1$-$C_6$-fluoroalcoxyle, cyano, $C(O)NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, aryle-$C_1$-$C_6$-alkylène, aryle ou hétéroaryle, l'aryle et l'hétéroaryle étant éventuellement substitués

par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$X_3$ et $X_4$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$, aryle-$C_1$-$C_6$-alkylène, aryle ou hétéroaryle, l'aryle et l'hétéroaryle étant éventuellement substitués par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$Z_1$, $Z_2$, $Z_3$, $Z_4$ représentent, indépendamment l'un de l'autre, un atome d'azote ou un groupe C(Re), l'un au moins correspondant à un atome d'azote et l'un au moins correspondant à un groupe $C(R_6)$ ; l'atome d'azote ou un des atomes d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ;

n est égal à 0, 1, 2 ou 3 ;

Y représente un aryle ou un hétéroaryle éventuellement substitué par un ou plusieurs groupes choisis parmi un atome d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, hydroxyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, cyano, $C(O)NR_1R_2$, nitro, $NR_1R_2$, $C_1$-$C_6$-thioalkyle, thiol, -S(O)-$C_1$-$C_6$-alkyle, -S $(O)_2$-$C_1$-$C_6$-alkyle, $SO_2NR_1R_2$, $NR_3COR_4$, $NR_3SO_2R_5$, aryle-$C_1$-$C_6$-alkylène ou aryle, l'aryle et l'aryle-$C_1$-$C_6$-alkylène étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

Z représente une amine cyclique reliée par l'atome d'azote, de formule:

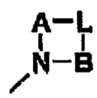

dans laquelle

A représente un groupe $C_1$-$C_7$-alkylène éventuellement substitué par un ou deux groupes $R_8$;
B représente un groupe $C_1$-$C_7$-alkylène éventuellement substitué par un ou deux groupes $R_9$ ;
L représente une liaison, un atome de soufre, d'oxygène ou d'azote ; l'atome d'azote étant éventuellement substitué par un groupe $R_{10}$ ou $R_{11}$.

les atomes de carbone de l'amine cyclique Z étant éventuellement substitués par un ou plusieurs groupes $R_{12}$ identiques ou différents l'un de l'autre ;

$R_1$ et $R_2$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle ; ou $R_1$ et $R_2$ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle ;

$R_3$ et $R_4$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle ;

$R_5$ représente un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle ou hétéroaryle ;

$R_6$ représente un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène,$C_1$-$C_6$-fluoroalkyle,$C_1$-$C_6$-alcoxyle,$C_3$-$C_7$-cycloalcoxyle,$C_3$-$C_7$-cycloalkyle-$C_1$-$C_6$-alkylène-O-, $C_1$-$C_6$-fluoroalcoxyle, $C_1$-$C_6$-thioalkyle, -S(O)-$C_1$-$C_6$-alkyle, -$S(O)_2$-$C_1$-$C_6$-alkyle, aryle, aryl-$C_1$-$C_6$-alkylène, hétéroaryle, hydroxyle, thiol, oxo ou thio ;

$R_7$ représente un atome d'hydrogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, aryle, aryl-$C_1$-$C_6$-alkylène ou hétéroaryle ;

$R_8$, $R_9$ et $R_{10}$ sont définis tels que :

deux groupes $R_8$ peuvent former ensemble une liaison ou un groupe $C_1$-$C_6$-alkylène ;
deux groupes $R_9$ peuvent former ensemble une liaison ou un groupe $C_1$-$C_6$-alkylène ;
$R_8$ et $R_9$ peuvent former ensemble une liaison ou un groupe $C_1$-$C_6$-alkylène ;
$R_8$ et $R_{10}$ peuvent former ensemble une liaison ou un groupe $C_1$-$C_6$-alkylène ;
$R_9$ et $R_{10}$ peuvent former ensemble une liaison ou un groupe $C_1$-$C_6$-alkylène ;

$R_{11}$ représente un atome d'hydrogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, hydroxyle, $COOR_6$, $C(O)NR_1R_2$, aryle-$C_1$-$C_6$-alkylène, aryle ou hétéroaryle, l'aryle et l'hétéroaryle étant éventuellement substitués par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;
$R_{12}$ représente un atome de fluor, un groupe $C_1$-$C_6$-alkyle éventuellement substitué par un groupe $R_{13}$, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-cycloalk-1,1-diyle, $C_3$-$C_7$-hétérocycloalk-1,1-diyle éventuellement substitué sur un atome d'azote par un groupe $R_{11}$ , $C_1$-$C_6$-alcoxyle, $C_3$-$C_7$-cycloalcoxyle, $C_3$-$C_7$-cycloalkyle-$C_1$-$C_6$-alkylène-O-, $C_1$-$C_6$-fluoroalcoxyle, $C(O)NR_1R_2$, $NR_1R_2$, $NR_3COR_4$, $OC(O)NR_1R_2$, $NR_3COOR_5$, $NR_3CONR_1R_2$, hydroxyle, thiol, oxo, thio, aryle-$C_1$-$C_6$-alkylène, aryle, l'aryle étant éventuellement substitué par un ou plusieurs substituants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_3$-$C_7$-cycloalcoxyle, $C_1$-$C_6$-fluoroalcoxyle, nitro ou cyano ;
$R_{13}$ représente un $C_1$-$C_6$-alcoxyle, $C_3$-$C_7$-cycloalcoxyle, $C_3$-$C_7$-cycloalkyle-$C_1$-$C_6$-alkylène-O-, $C(O)NR_1R_2$, $NR_1R_2$, $NR_3COR_4$, $OC(O)NR_1R_2$, $NR_3COOR_5$, hydroxyle ;

le ou les atomes d'azote du composé de formule générale (I) pouvant être sous forme oxydée;
le ou les atomes de soufre du composé de formule générale (I) pouvant être sous forme oxydée ;
à l'état de base ou de sel d'addition à un acide, ainsi qu'à l'état d'hydrate ou de solvat.

**2.** Composé de formule (I) selon la revendication 1, **caractérisé en ce que** $X_1$, $X_2$, $X_3$ et $X_4$ sont choisis, indépendamment l'un de l'autre, parmi un atome d'hydrogène ou d'halogène ou un groupe $C_1$-$C_6$-alkyle, $C_1$-$C_6$-fluoroalkyle, $C_1$-$C_6$-thioalkyle, $-S(O)_2$-$C_1$-$C_6$-alkyle ; à l'état de base ou de sel d'addition à un acide, ainsi qu'à l'état d'hydrate ou de solvat.

**3.** Composé de formule (I) selon la revendication 1, **caractérisé en ce que**
$X_1$ représente un atome un atome d'hydrogène ou d'halogène ou un groupe $C_1$-$C_6$-alkyle ;
$X_2$ représente un atome d'hydrogène ou d'halogène ou un groupe $C_1$-$C_6$-alkyle, $C_1$-$C_6$-fluoroalkyle, $-S(O)_2$-$C_1$-$C_6$-alkyle ;
$X_3$ et $X_4$ représentent, indépendamment l'un de l'autre, un atome d'hydrogène ou d'halogène ou un groupe $C_1$-$C_6$-alkyle, $C_1$-$C_6$-fluoroalkyle, $C_1$-$C_6$-alcoxyle, $NR_1R_2$, $C_1$-$C_6$-thioalkyle ;
$R_1$ et $R_2$ représentent, indépendamment l'un de l'autre, un groupe $C_1$-$C_6$-alkyle ;
à l'état de base ou de sel d'addition à un acide, ainsi qu'à l'état d'hydrate ou de solvat.

**4.** Composé de formule (I) selon l'une quelconque des revendications 1 à 3, **caractérisé en ce que** $Z_1$, $Z_2$, $Z_3$, $Z_4$ représentent, indépendamment l'un de l'autre, un atome d'azote ou un groupe $C(R_6)$, deux d'entre eux au moins correspondant à un groupe $C(R_6)$ ; l'atome d'azote ou un des atomes d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ; $R_6$ et $R_7$ étant tels que définis dans la formule générale (I) selon la revendication 1 ;
à l'état de base ou de sel d'addition à un acide, ainsi qu'à l'état d'hydrate ou de solvat.

**5.** Composé de formule (I) selon la revendication 4, **caractérisé en ce que** $Z_1$ et $Z_3$ représentent un groupe $C(R_6)$ et $Z_2$ et $Z_4$ représentent un atome d'azote; $R_6$ correspondant à un atome d'hydrogène ; à l'état de base ou de sel d'addition à un acide, ainsi qu'à l'état d'hydrate ou de solvat.

**6.** Composé de formule (I) selon la revendication 4, **caractérisé en ce que** $Z_1$, $Z_2$, $Z_3$, $Z_4$ représentent, indépendamment l'un de l'autre, un atome d'azote ou un groupe $C(R_6)$, l'un correspondant à un atome d'azote et tes autres correspondant à un groupe $C(R_6)$ ; l'atome d'azote présent dans le cycle, défini comme azote de position 1, étant éventuellement substitué par $R_7$ lorsque l'atome de carbone en position 2 ou 4 par rapport à cet azote de référence est substitué par un groupe oxo ou thio ; $R_6$ et $R_7$ étant tels que définis dans la formule générale (I) selon la revendication

1 ; à l'état de base ou de sel d'addition à un acide, ainsi qu'à l'état d'hydrate ou de solvat.

**7.** Composé de formule (I) selon la revendication 6, **caractérisé en ce que** $Z_4$ représente un atome d'azote et $Z_1$, $Z_2$ et $Z_3$ représentent, indépendamment l'un de l'autre, un groupe $C(R_6)$ ; $R_6$ représente un atome d'hydrogène ou un groupe $C_1$-$C_6$-alkyle, $C_1$-$C_6$-fluoroalkyle ou $C_1$-$C_6$-alcoxyle ; à l'état de base ou de sel d'addition à un acide, ainsi qu'à l'état d'hydrate ou de solvat.

**8.** Composé de formule (I) selon l'une quelconque des revendications 1 à 7, **caractérisé en ce que** n est égal à 1 ; à l'état de base ou de sel d'addition à un acide, ainsi qu'à l'état d'hydrate ou de solvat.

**9.** Composé de formule (I) selon l'une quelconque des revendications 1 à 8, **caractérisé en ce que** Y représente un aryle ou un hétéroaryle éventuellement substitué par un ou plusieurs groupes choisis parmi un atome d'halogène ou un groupe $C_1$-$C_6$-alkyle ou $NR_1R_2$ :
$R_1$ et $R_2$ forment ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe $C_1$-$C_6$-alkyle, $C_3$-$C_7$-cycloalkyle, $C_3$-$C_7$-cycloalkyle-C1-$C_3$-alkylène, aryle-$C_1$-$C_6$-alkylène, aryle ou hétéroaryle ;
à l'état de base ou de sel d'addition à un acide, ainsi qu'à l'état d'hydrate ou de solvat.

**10.** Composé de formule (I) selon l'une quelconque des revendications 1 à 9, **caractérisé en ce que** Z représente une amine cyclique reliée par l'atome d'azote, de formule :

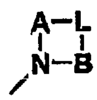

dans laquelle

A représente un groupe $C_1$-$C_4$-alkylène_éventuellement substitué par un ou deux groupes $R_6$;
B représente un groupe $C_1$-$C_4$-alkylène éventuellement substitué par un ou deux groupes $R_9$;
L représente une liaison ou un atome d'oxygène ;

l'atome d'azote de l'amine cyclique Z pouvant être sous forme N-oxyde ;
les atomes de carbone de l'amine cyclique Z étant éventuellement substitués par un groupe $R_{12}$ ;
$R_8$ et $R_9$ sont définis tels que :

deux groupes $R_8$ peuvent former ensemble une liaison ; ou
deux groupes $R_9$ peuvent former ensemble une liaison ; ou
$R_8$ et $R_9$ peuvent former ensemble une liaison ;

$R_{12}$ représente un groupe $NR_1R_2$, $NR_3COOR_5$, hydroxyle ;
$R_1$ et $R_2$, représentent, indépendamment l'un de l'autre, un atome d'hydrogène ;
$R_3$ représente un atome d'hydrogène ;
$R_5$ représente un groupe $C_1$-$C_6$-alkyle ;
à l'état de base ou de sel d'addition à un acide, ainsi qu'à l'état d'hydrate ou de solvat.

**11.** Composé de formule (I) selon la revendication 10, **caractérisé en ce que** Z représente une amine cyclique choisie parmi l'azétidine, la pyrrolidine, la pipéridine, la morpholine, l'azabicyclo [3.1.0]hexane et l'azabicyclo[3.2.0]heptane ;
l'atome d'azote de l'amine cyclique Z pouvant être sous forme N-oxyde ;
les atomes de carbone de l'amine cyclique Z étant éventuellement substitués par un groupe $R_{12}$ ;
$R_{12}$ représente un groupe $NR_1R_2$, $NR_3COOR_5$, hydroxyle ;
$R_1$ et $R_2$, représentent, indépendamment l'un de l'autre, un atome d'hydrogène ;
$R_3$ représente un atome d'hydrogène ;
$R_5$ représente un groupe $C_1$-$C_6$-alkyle ;
à l'état de base ou de sel d'addition à un acide, ainsi qu'à l'état d'hydrate ou de solvat.

**12.** Composé de formule (I) selon la revendication 11, **caractérisé en ce que** Z représente une amine cyclique choisie parmi l'azétidine, la pyrrolidine, la pipéridine, la morpholine, l'azabicylo [3.1.0]hexane et l'azabicylo[3.2.0]heptane ;
les atomes de carbone de l'azétidine étant éventuellement substitués par un groupe hydroxyle ;
les atomes de carbone de la pyrrolidine étant éventuellement substitués par un groupe $NR_1R_2$, $NR_3COOR_5$, hydroxyle ; l'atome d'azote de la pyrrolidine pouvant être sous forme N-oxyde ;
$R_1$ et $R_2$, représentent, indépendamment l'un de l'autre, un atome d'hydrogène;
$R_3$ représente un atome d'hydrogène ;
$R_5$ représente un groupe $C_1$-$C_6$-alkyle ;
à l'état de base ou de sel d'addition à un acide, ainsi qu'à l'état d'hydrate ou de solvat.

**13.** Procédé de préparation d'un composé de formule (I) selon l'une quelconque des revendications 1 à 12, **caractérisé en ce que** l'on fait réagir un composé de formule générale (IV)

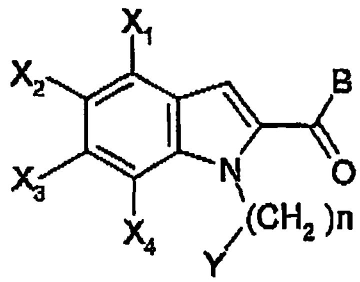

(IV)

dans laquelle $X_1$, $X_2$, $X_3$, $X_4$, Y et n sont tels que définis dans la formule générale (I) selon la revendication 1 et B représente un atome de chlore,
avec une amine de formule générale (V)

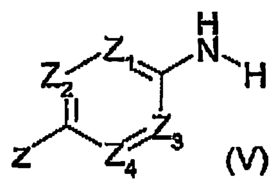

(V)

dans laquelle $Z_1$, $Z_2$, $Z_3$, $Z_4$ et Z sont tels que définis dans la formule générale (I) selon la revendication 1, dans un solvant.

**14.** Procédé de préparation d'un composé de formule (I) selon l'une quelconque des revendications 1 à 12, **caractérisé en ce que** l'on fait réagir un composé de formule générale (IV)

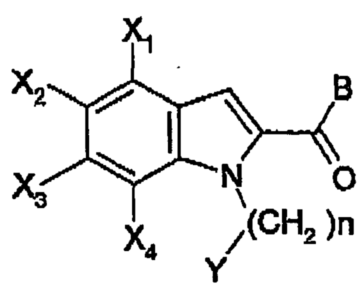

(IV)

dans laquelle $X_1$, $X_2$, $X_3$, $X_4$, Y et n sont tels que définis dans la formule générale (I) selon la revendication 1 et B représente un groupe hydroxyle,
avec une amine de formule générale (V)

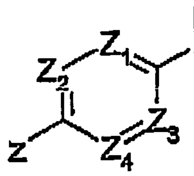

(V)

dans laquelle $Z_1$, $Z_2$, $Z_3$, $Z_4$ et Z sont tels que définis dans la formule générale (I) selon la revendication 1, en présence d'un agent de couplage et d'une base dans un solvant.

**15.** Procédé de préparation d'un composé de formule (I) selon l'une quelconque des revendications 1 à 12, **caractérisé en ce que** l'on fait réagir un composé de formule générale M)

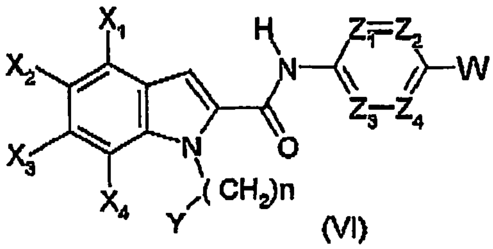

(VI)

dans laquelle $X_1$, $X_2$, $X_3$, $X_4$, $Z_1$, $Z_2$, $Z_3$, $Z_4$, Y et n sont tels que définis dans la formule générale (I) selon la revendication 1 et W représente un atome d'halogène, en présence d'une amine de formule ZH dans laquelle Z est une amine cyclique telle que définie dans la formule générale (I) selon la revendication 1.

**16.** Amine de formule générale (V) choisie parmi les amines suivantes :

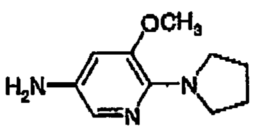

(Va)

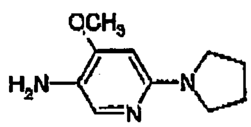

(Vb)

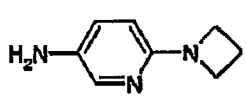

(Vc)

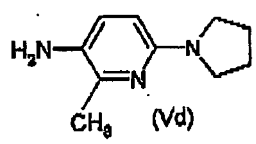

(Vd)

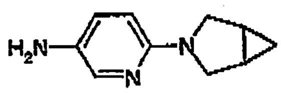

(Vf)

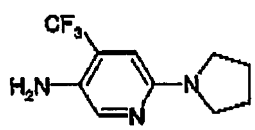

(Vg)

à l'état de base ou de sel d'addition à un acide.

**17.** Médicament, **caractérisé en ce qu'**il comprend un composé de formule (I), selon l'une quelconque des revendications 1 à 12, ou un sel pharmaceutiquement acceptable, ou encore un hydrate ou un solvat du composé de formule (I).

**18.** Composition pharmaceutique, **caractérisée en ce qu'**elle comprend un composé de formule (I), selon l'une quelconque des revendications 1 à 12, ou un sel pharmaceutiquement acceptable, un hydrate ou un solvat de ce composé, ainsi qu'au moins un excipient pharmaceutiquement acceptable.

**19.** Utilisation d'un composé de formule (I), selon l'une quelconque der revendications 1 à 12, pour la préparation d'un médicament destiné à prévenir ou à traiter les pathologies dans lesquelles les récepteurs de type TRPV1 sont

impliqués.

**20.** Utilisation d'un composé de formule (1), selon l'une quelconque des revendications 1 à 12, pour la préparation d'un médicament destiné à prévenir ou à traiter la douleur, l'inflammation, les désordres urologiques, les désordres gynécologiques, les désordres gastrointestinaux, des désordres respiratoires, le psoriasis, le pruritis, les irritations dermiques, des yeux ou des muqueuses, l'herpès, le zona, ou à traiter la dépression ou le diabète.

## Claims

**1.** Compound corresponding to formula (I)

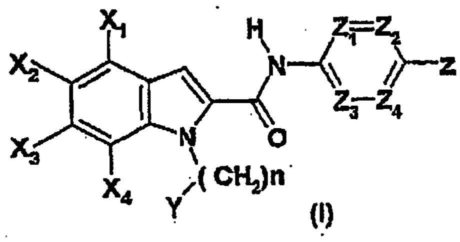

(I)

in which

$X_1$ is a hydrogen or halogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, cyano, $C(O)NR_1R_2$, nitro, $C_1$-$C_6$-thioalkyl, -S(O)-$C_1$-$C_6$-alkyl, -$S(O)_2$-$C_1$-$C_6$-alkyl, $SO_2NR_1R_2$, aryl-$C_1$-$C_6$-alkylene, aryl or heteroaryl group, the aryl and heteroaryl being optionally substituted with one or more substituents selected from a halogen, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, nitro or cyano group;

$X_2$ is a hydrogen or halogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_3$-$C_7$-cycloalkoxyl, $C_3$-$C_7$-cycloalkyl-$C_1$-$C_6$-alkylene-O-, $C_1$-$C_6$-fluoroalkoxyl, cyano, $C(O)NR_1R_2$, $C_1$-$C_6$-thioalkyl, -S(O)-$C_1$-$C_6$-alkyl, -$S(O)_2$-$C_1$-$C_6$-alkyl, $SO_2NR_1R_2$, aryl-$C_1$-$C_6$-alkylene, aryl or heteroaryl group, the aryl and the heteroaryl being optionally substituted with one or more substituents selected from a halogen, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, nitro or cyano group;

$X_3$ and $X_4$ are, independently of one another, a hydrogen or halogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_3$-$C_7$-cycloalkoxyl, $C_3$-$C_7$-cycloalkyl-$C_1$-$C_6$-alkylene-O-, $C_1$-$C_6$-fluoroalkoxyl, cyano, $C(O)NR_1R_2$, nitro, $NR_1R_2$, $C_1$-$C_6$-thioalkyl, -S(O)-$C_1$-$C_6$-alkyl,-S$(O)_2$-$C_1$-$C_6$-alkyl, $SO_2NR_1R_2$, $NR_3COR_4$, $NR_3SO_2R_5$, aryl-$C_1$-$C_6$-alkylene, aryl or heteroaryl group, the aryl and the heteroaryl being optionally substituted with one or more substituents selected from a halogen, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, nitro or cyano group;

$Z_1$, $Z_2$, $Z_3$ and $Z_4$ are, independently of one another, a nitrogen atom or a $C(R_6)$ group, at least one corresponding to a nitrogen atom and at least one corresponding to a $C(R_6)$ group; the nitrogen atom or one of the nitrogen atoms present in the ring, defined as nitrogen in the 1-position, being optionally substituted with $R_7$ when the carbon atom in the 2- or 4-position with respect to this reference nitrogen is substituted with an oxo or thio group;

n is equal to 0, 1, 2 or 3;

Y is an aryl or a heteroaryl optionally substituted with one or more groups selected from a halogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, hydroxyl, $C_1$-$C_6$-alkoxyl, $C_3$-$C_7$-cycloalkoxyl, $C_3$-$C_7$-cycloalkyl-$C_1$-$C_6$-alkylene-O-, $C_1$-$C_6$-fluoroalkoxyl, cyano, $C(O)NR_1R_2$, nitro, $NR_1R_2$, $C_1$-$C_6$-thioalkyl, thiol, -S(O)-$C_1$-$C_6$-alkyl, -$S(O)_2$-$C_1$-$C_6$-alkyl, $SO_2NR_1R_2$, $NR_3COR_4$, $NR_3SO_2R_5$, aryl-$C_1$-$C_6$-alkylene or aryl group, the aryl and the aryl-$C_1$-$C_6$-alkylene being optionally substituted with one or more substituents selected from a halogen, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, nitro or cyano group;

Z is a cyclic amine attached via the nitrogen atom, of formula:

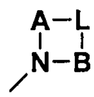

in which

A is a $C_1$-$C_7$-alkylene group optionally substituted with one or two groups $R_8$;
B is a $C_1$-$C_7$-alkylene group optionally substituted with one or two groups $R_9$;
L is a bond, or a sulphur, oxygen or nitrogen atom; the nitrogen atom being optionally substituted with a group $R_{10}$ or $R_{11}$,

the carbon atoms of the cyclic amine Z being optionally substituted with one or more groups $R_{12}$ which may be identical to or different from one another;
$R_1$ and $R_2$ are, independently of one another, a hydrogen atom or a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene, aryl or heteroaryl group; or $R_1$ and $R_2$ form, together with the nitrogen atom which bears them, an azetidinyl, pyrrolidinyl, piperidinyl, azepinyl, morpholinyl, thiomorpholinyl, piperazinyl or homopiperazinyl group, this group being optionally substituted with a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene, aryl or heteroaryl group;
$R_3$ and $R_4$ are, independently of one another, a hydrogen atom or a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene, aryl or heteroaryl group;
$R_5$ is a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene, aryl or heteroaryl group;
$R_6$ is a hydrogen or halogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_3$-$C_7$-cycloalkoxyl, $C_3$-$C_7$-cycloalkyl-$C_1$-$C_6$-alkylene-O-, $C_1$-$C_6$-fluoroalkoxyl, $C_1$-$C_6$-thioalkyl, -S(O)-$C_1$-$C_6$-alkyl, -$S(O)_2$-$C_1$-$C_6$-alkyl, aryl, aryl-$C_1$-$C_6$-alkylene, heteroaryl, hydroxyl, thiol, oxo or thio group;
$R_7$ is a hydrogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_3$-$C_7$-cycloalkoxyl, $C_3$-$C_7$-cycloalkyl-$C_1$-$C_6$-alkylene-O-, $C_1$-$C_6$-fluoroalkoxyl, aryl, aryl-$C_1$-$C_6$-alkylene or heteroaryl group;
$R_8$, $R_9$ and $R_{10}$ are defined such that:

two groups $R_8$ can together form a bond or a $C_1$-$C_6$-alkylene group;
two groups $R_9$ can together form a bond or a $C_1$-$C_6$-alkylene group;
$R_8$ and $R_9$ can together form a bond or a $C_1$-$C_6$-alkylene group;
$R_8$ and $R_{10}$ can together form a bond or a $C_1$-$C_6$-alkylene group;
$R_9$ and $R_{10}$ can together form a bond or a $C_1$-$C_6$-alkylene group;

$R_{11}$ is a hydrogen atom or a $C_1$-$C_6$-alkyl, $C_3$-$C_7$-cyclalkyl, $C_3$-$C_7$-cycloalkyl-$C_1$-$C_3$-alkylene, $C_1$-$C_6$-fluoroalkyl, $C_1$-$C_6$-alkoxyl, $C_3$-$C_7$-cycloalkoxyl, $C_3$-$C_7$-cycloalkyl-$C_1$-$C_6$-alkylene-O-, $C_1$-$C_6$-fluoroalkoxyl, hydroxyl, $COOR_5$, $C(O)NR_1R_2$, aryl-$C_1$-$C_6$-alkylene, aryl or heteroaryl group, the aryl and the heteroaryl being optionally substituted with one or more substituents selected from a halogen, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_3$-$C_7$-cycloalkoxyl, $C_1$-$C_6$-fluoroalkoxyl, nitro or cyano group;
$R_{12}$ is a fluorine atom, a $C_1$-$C_6$-alkyl group optionally substituted with a group $R_{13}$, a $C_3$-$C_7$-cycloalkyl, $C_3$-$C_7$-cycloalkyl-$C_1$-$C_3$-alkylene, $C_1$-$C_6$-fluoroalkyl or $C_l$-$C_6$-cycloalk-1,1-diyl group, a $C_3$-$C_7$-heterocycloalk-1,1-diyl group optionally substituted on a nitrogen atom with a group $R_{11}$, or a $C_1$-$C_6$-alkoxyl, $C_3$-$C_7$-cycloalkoxyl, $C_3$-$C_7$-cycloalkyl-$C_1$-$C_6$-alkylene-O-, $C_1$-$C_6$-fluoroalkoxyl, $C(O)NR_1R_2$, $NR_1R_2$, $NR_3COR_4$, $OC(O)NR_1R_2$, $NR_3COOR_5$, $NR_3CONR_1R_2$, hydroxyl, thiol, oxo, thio, aryl-$C_1$-$C_6$-alkylene or aryl group, the aryl being optionally substituted with one or more substituents selected from a halogen, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_3$-$C_7$-cycloalkoxyl, $C_1$-$C_6$-fluoroalkoxyl, nitro or cyano group;
$R_{13}$ is a $C_1$-$C_6$-alkoxyl, $C_3$-$C_7$-cycloalkoxyl, $C_3$-$C_7$-cycloalkyl-$C_1$-$C_6$-alkylene-O-, $C(O)NR_1R_2$, $NR_1R_2$, $NR_3COR_4$, $OC(O)NR_1R_2$, $NR_3COOR_5$ or hydroxyl;

it being possible for the nitrogen atom(s) of the compound of formula (I) to be in oxidized form;
it being possible for the sulphur atom(s) of the compound of formula (I) to be in oxidized form;
in the form of a base or of an addition salt with an acid, and also in the form of a hydrate or of a solvate.

**2.** Compound of formula (I) according to Claim 1, **characterized in that** $X_1$, $X_2$, $X_3$ and $X_4$ are selected, independently

of one another, from a hydrogen or halogen atom or a $C_1$-$C_6$-alkyl, $C_1$-$C_6$-fluoroalkyl, $C_1$-$C_6$-thioalkyl or -S$(O)_2$-$C_1$-$C_6$-alkyl group;
in the form of a base or of an addition salt with an acid, and also in the form of a hydrate or of a solvate.

**3.** Compound of formula (I) according to Claim 1, **characterized in that**
$X_1$ is a hydrogen or halogen atom or a $C_1$-$C_6$-alkyl group;
$X_2$ is a hydrogen or halogen atom or a $C_1$-$C_6$-alkyl, $C_1$-$C_6$-fluoroalkyl or -$S(O)_2$-$C_1$-$C_6$-alkyl group;
$X_3$ and $X_4$ are, independently of one another, a hydrogen or halogen atom or a $C_1$-$C_6$-alkyl, $C_1$-$C_6$-fluoroalkyl, $C_1$-$C_6$-alkoxyl, $NR_1R_2$ or $C_1$-$C_6$-thioalkyl group;
$R_1$ and $R_2$ are, independently of one another, a $C_1$-$C_6$-alkyl group;
in the form of a base or of an addition salt with an acid, and also in the form of a hydrate or of a solvate.

**4.** Compound of formula (I) according to any one of Claims 1 to 3, **characterized in that** $Z_1$, $Z_2$, $Z_3$ and $Z_4$ are, independently of one another, a nitrogen atom or a $C(R_6)$ group, at least two of them corresponding to a $C(R_6)$ group; the nitrogen atom or one of the nitrogen atoms present in the ring, defined as nitrogen in the 1-position, being optionally substituted with $R_7$ when the carbon atom in the 2- or 4-position with respect to this reference nitrogen is substituted with an oxo or thio group; $R_6$ and $R_7$ being as defined in formula (I) according to Claim 1;
in the form of a base or of an addition salt with an acid, and also in the form of a hydrate or of a solvate.

**5.** Compound of formula (I) according to Claim 4, **characterized in that** $Z_1$ and $Z_3$ are a $C(R_6)$ group and $Z_2$ and $Z_4$ are a nitrogen atom; $R_6$ corresponding to a hydrogen atom; in the form of a base or of an addition salt with an acid, and also in the form of a hydrate or of a solvate.

**6.** Compound of formula (I) according to Claim 4, **characterized in that** $Z_1$, $Z_2$, $Z_3$ and $Z_4$ are, independently of one another, a nitrogen atom or a $C(R_6)$ group, one corresponding to a nitrogen atom and the others corresponding to a $C(R_6)$ group; the nitrogen atom present in the cycle, defined as nitrogen in the 1-position, being optionally substituted with $R_7$ when the carbon atom in the 2- or 4-position with respect to this reference nitrogen is substituted with an oxo or thio group; $R_6$ and $R_7$ being as defined in formula (I) according to Claim 1;
in the form of a base or of an addition salt with an acid, and also in the form of a hydrate or of a solvate.

**7.** Compound of formula (I) according to Claim 6, **characterized in that**
$Z_4$ is a nitrogen atom and $Z_1$, $Z_2$ and $Z_3$ are, independently of one another, a $C(R_6)$ group;
$R_6$ is a hydrogen atom or a $C_1$-$C_6$-alkyl, $C_1$-$C_6$-fluoroalkyl or $C_1$-$C_6$-alkoxyl group;
in the form of a base or of an addition salt with an acid, and also in the form of a hydrate or of a solvate.

**8.** Compound of formula (I) according to any one of Claims 1 to 7, **characterized in that** n is equal to 1;
in the form of a base or of an addition salt with an acid, and also in the form of a hydrate or of a solvate.

**9.** Compound of formula (I) according to any one of Claims 1 to 8, **characterized in that** Y is an aryl or a heteroaryl optionally substituted with one or more groups selected from a halogen atom or a $C_1$-$C_6$-alkyl or $NR_1R_2$ group;
$R_1$ and $R_2$ form, together with the nitrogen atom which bears them, an azetidinyl, pyrrolidinyl, piperidinyl, azepinyl, morpholinyl, thiomorpholinyl, piperazinyl or homopiperazinyl group, this group being optionally substituted with a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene, aryl or heteroaryl group;
in the form of a base or of an addition salt with an acid, and also in the form of a hydrate or of a solvate.

**10.** Compound of formula (I) according to any one of Claims 1 to 9, **characterized in that** Z is a cyclic amine attached via the nitrogen atom, of formula:

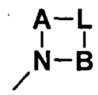

in which

A is a $C_1$-$C_4$-alkylene group optionally substituted with one or two groups $R_8$;
B is a $C_1$-$C_4$-alkylene group optionally substituted with one or two groups $R_9$;
L is a bond or an oxygen atom;

it being possible for the nitrogen atom of the cyclic amine Z to be in N-oxide form;
the carbon atoms of the cyclic amine Z being optionally substituted with a group $R_{12}$;
$R_8$ and $R_9$ are defined such that:

- two groups $R_8$ can together form a bond; or
- two groups $R_9$ can together form a bond; or
- $R_8$ and $R_9$ can together form a bond;

$R_{12}$ is an $NR_1R_2$, $NR_3COOR_5$ or hydroxyl group;
$R_1$ and $R_2$ are, independently of one another, a hydrogen atom;
$R_3$ is a hydrogen atom;
$R_5$ is a $C_1$-$C_6$-alkyl group;
in the form of a base or of an addition salt with an acid, and also in the form of a hydrate or of a solvate.

**11.** Compound of formula (I) according to Claim 10, **characterized in that**
Z is a cyclic amine selected from azetidine, pyrrolidine, piperidine, morpholine, azabicyclo[3.1.0]hexane and azabicyclo[3.2.0]heptane;
it being possible for the nitrogen atom of the cyclic amine Z to be in N-oxide form;
the carbon atoms of the cyclic amine Z being optionally substituted with a group $R_{12}$;
$R_{12}$ is an $NR_1R_2$, $NR_3COOR_5$ or hydroxyl group;
$R_1$ and $R_2$ are, independently of one another, a hydrogen atom;
$R_3$ is a hydrogen atom;
$R_5$ is a $C_1$-$C_6$-alkyl group;
in the form of a base or of an addition salt with an acid, and also in the form of a hydrate or of a solvate.

**12.** Compound of formula (I) according to Claim 11, **characterized in that**
Z is a cyclic amine selected from azetidine, pyrrolidine, piperidine, morpholine, azabicyclo[3.1.0]hexane and azabicyclo[3.2.0]heptane;
the carbon atoms of the azetidine being optionally substituted with a hydroxyl group;
the carbon atoms of the pyrrolidine being optionally substituted with an $NR_1R_2$, $NR_3COOR_5$ or hydroxyl group; it being possible for the nitrogen atom of the pyrrolidine to be in N-oxide form;
$R_1$ and $R_2$ are, independently of one another, a hydrogen atom;
$R_3$ is a hydrogen atom;
$R_5$ is a $C_1$-$C_6$-alkyl group;
in the form of a base or of an addition salt with an acid, and also in the form of a hydrate or of a solvate.

**13.** Process for preparing a compound of formula (I) according to any one of Claims 1 to 12, **characterized in that** a compound of formula (IV)

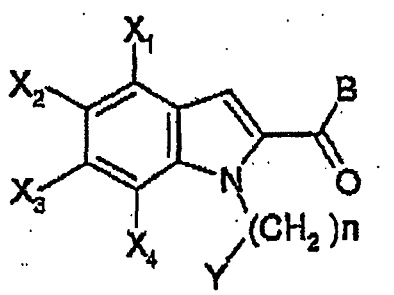

(IV)

in which $X_1$, $X_2$, $X_3$, $X_4$, Y and n are as defined in formula (I) according to Claim 1 and B is a chlorine atom,
is reacted with an amine of formula (V)

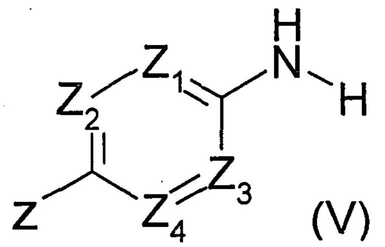

(V)

in which $Z_1$, $Z_2$, $Z_3$, $Z_4$ and Z are as defined in formula (I) according to Claim 1, in a solvent.

**14.** Process for preparing a compound of formula (I) according to any one of Claims 1 to 12, **characterized in that** a compound of formula (IV)

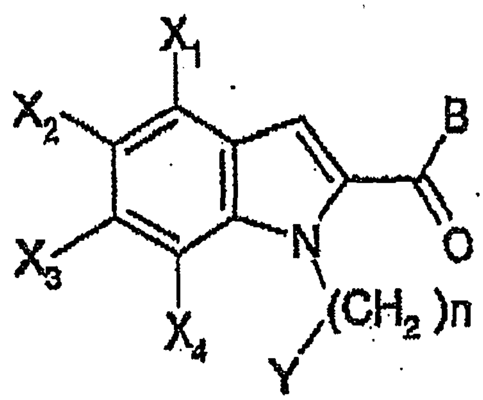

(IV)

in which $X_1$, $X_2$, $X_3$, $X_4$, Y and n are as defined in formula (I) according to Claim 1 and B is a hydroxyl group, is reacted with an amine of formula (V)

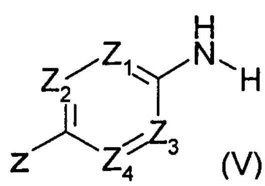

(V)

in which $Z_1$, $Z_2$, $Z_3$, $Z_4$ and Z are as defined in formula (I) according to Claim 1, in the presence of a coupling agent and of a base in a solvent.

**15.** Process for preparing a compound of formula (I) according to any one of Claims 1 to 12, **characterized in that** a compound of formula (VI)

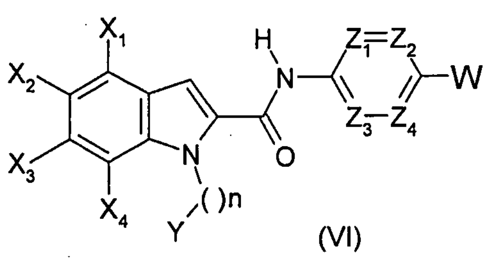

(VI)

in which $X_1$, $X_2$, $X_3$, $X_4$, $Z_1$, $Z_2$, $Z_3$, $Z_4$, Y and n are as defined in formula (I) according to Claim 1 and W is a halogen atom, is reacted in the presence of an amine of formula ZH in which Z is a cyclic amine as defined in formula (I)

according to Claim 1.

**16.** Amine of formula (V) selected from the following amines:

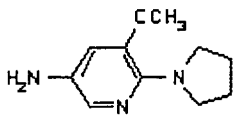
(Va)

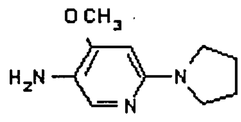
(Vb)

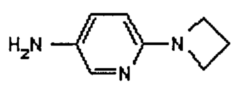
(Vc)

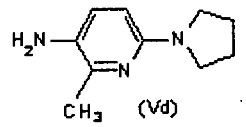
(Vd)

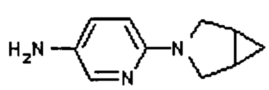
(Vf)

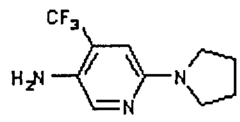
(Vg)

in the form of a base or of an addition salt with an acid.

**17.** Medicament, **characterized in that** it comprises a compound of formula (I) according to any one of Claims 1 to 12, or a pharmaceutically acceptable salt, or else a hydrate or a solvate of the compound of formula (I).

**18.** Pharmaceutical composition, **characterized in that** it comprises a compound of formula (I) according to any one of Claims 1 to 12, or a pharmaceutically acceptable salt, a hydrate or a solvate of this compound, and also at least one pharmaceutically acceptable excipient.

**19.** Use of a compound of formula (I) according to any one of Claims 1 to 12, for the preparation of a medicament for preventing or treating pathologies in which TRPV1-type receptors are involved.

**20.** Use of a compound of formula (I) according to any one of Claims 1 to 12, for the preparation of a medicament for preventing or treating pain, inflammation, urological disorders, gynaecological disorders, gastrointestinal disorders, respiratory disorders, psoriasis, pruritis, dermal, ocular or mucosal irritations, herpes or shingles, or for treating depression or diabetes.

**Patentansprüche**

**1.** Verbindung der Formel (I)

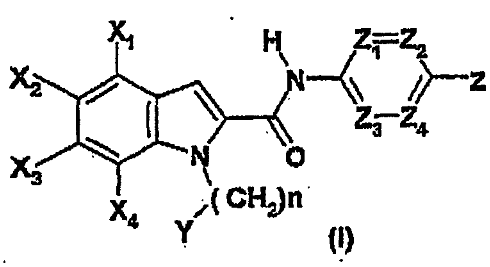
(I)

worin

$X_1$ für ein Wasserstoff- oder Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Cyano-, $C(O)NR_1R_2$-, Nitro-, $C_1$-$C_6$-Thioalkyl-, -S(O)-$C_1$-$C_6$-Alkyl-, -$S(O)_2$-$C_1$-$C_6$-Alkyl-, $SO_2NR_1R_2$-, Aryl-$C_1$-$C_6$-alkylen-, Aryl- oder Heteroarylgruppe steht, wobei das Aryl und das Heteroaryl gegebenenfalls durch einen oder mehrere unter einem Halogen und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-, Nitro- oder Cyano-

gruppe ausgewählte Substituenten substituiert sind;

$X_2$ für ein Wasserstoff- oder Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_3$-$C_7$-Cycloalkoxy-, $C_3$-$C_7$-Cycloalkyl-$C_1$-$C_3$-alkylen-O-, $C_1$-$C_6$-Fluoralkoxy-, Cyano-, C(O)$NR_1R_2$-, $C_1$-$C_6$-Thioalkyl-, -S(O)-$C_1$-$C_6$-Alkyl-,- $S(O)_2$-$C_1$-$C_6$-Alkyl-, $SO_2NR_1R_2$-, Aryl-$C_1$-$C_6$-alkylen-, Aryl- oder Heteroarylgruppe steht, wobei das Aryl und das Heteroaryl gegebenenfalls durch einen oder mehrere unter einem Halogen und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-, Nitro- oder Cyanogruppe ausgewählte Substituenten substituiert sind;

$X_3$ und $X_4$ unabhängig voneinander für ein Wasserstoff- oder Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_3$-$C_7$-Cycloalkoxy-, $C_3$-$C_7$-Cycloalkyl-$C_1$-$C_6$-alkylen-O-, $C_1$-$C_6$-Fluoralkoxy-, Cyano-, C(O)$NR_1R_2$-, Nitro-, $NR_1R_2$-, $C_1$-$C_6$-Thioalkyl-, -S (O)-$C_1$-$C_6$-Alkyl-, -$S(O)_2$-$C_1$-$C_6$-Alkyl-, $SO_2NR_1R_2$-, $NR_3COR_4$-, $NR_3SO_2R_5$-, Aryl-$C_1$-$C_6$-alkylen-, Aryl- oder Heteroarylgruppe stehen, wobei das Aryl und das Heteroaryl gegebenenfalls durch einen oder mehrere unter einem Halogen und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-" $C_1$-$C_6$-Fluoralkoxy-, Nitro- oder Cyanogruppe ausgewählte Substituenten substituiert sind;

$Z_1$, $Z_2$, $Z_3$ und $Z_4$ unabhängig voneinander für ein Stickstoffatom oder eine C($R_6$)-Gruppe stehen, wobei mindestens eine der Variablen für ein Stickstoffatom und mindestens eine der Variablen für eine C($R_6$)-Gruppe steht; wobei das Stickstoffatom bzw. die Stickstoffatome im Ring, definiert als Stickstoff in der 1-Position, gegebenenfalls durch $R_7$ substituiert ist bzw. sind, wenn das Kohlenstoffatom in 2- oder 4-Position zu diesem Referenzstickstoff durch eine Oxo- oder Thiogruppe substituiert ist;

n gleich 0, 1, 2 oder 3 ist;

Y für ein Aryl oder Heteroaryl steht, das gegebenenfalls durch eine oder mehrere unter einem Halogenatom oder einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Hydroxyl-, $C_1$-$C_6$-Alkoxy, $C_3$-$C_7$-Cycloalkoxy-, $C_3$-$C_7$-Cycloalkyl-$C_1$-$C_6$-alkylen-O-, $C_1$-$C_6$-Fluoralkoxy-, Cyano-, C(O) $NR_1R_2$-, Nitro-, $NR_1R_2$-, $C_1$-$C_6$-Thioalkyl-, Thiol-, -S(O)-$C_1$-$C_6$-Alkyl-, -$S(O)_2$-$C_1$-$C_6$-Alkyl, $SO_2NR_1R_2$-, $NR_3COR_4$-, $NR_3SO_2R_5$-, Aryl-$C_1$-$C_6$-alkylen- oder Arylgruppe ausgewählte Gruppen substituiert ist, wobei das Aryl und das Aryl-$C_1$-$C_6$-alkylen gegebenenfalls durch einen oder mehrere unter einem Halogen und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-, Nitro- oder Cyanogruppe ausgewählte Substituenten substituiert sind;

Z für ein über das Stickstoffatom gebundenes cyclisches Amin der Formel:

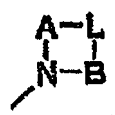

steht, worin

A für eine gegebenenfalls durch eine oder zwei Gruppen $R_8$ substituierte $C_1$-$C_7$-Alkylengruppe steht;
B für eine gegebenenfalls durch eine oder zwei Gruppen $R_9$ substituierte $C_1$-$C_7$-Alkylen-gruppe steht;
L für eine Bindung oder ein Schwefel-, Sauerstoff- oder Stickstoffatom steht; wobei das Stickstoffatom gegebenenfalls durch eine Gruppe $R_{10}$ oder $R_{11}$ substituiert ist;

wobei die Kohlenstoffatome des cyclischen Amins Z gegebenenfalls durch eine oder mehrere gleiche oder voneinander verschiedene Gruppen $R_{12}$ substituiert sind;

$R_1$ und $R_2$ unabhängig voneinander für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen-, Aryl- oder Heteroarylgruppe stehen oder $R_1$ und $R_2$ gemeinsam mit dem Stickstoffatom, an das sie gebunden sind, eine Azetidinyl-, Pyrrolidinyl-, Piperidinyl-, Azepinyl-, Morpholinyl-, Thiomorpholinyl-, Piperazinyl- oder Homopiperazinylgruppe bilden, wobei diese Gruppe gegebenenfalls durch eine $C_1$-$C_6$-Alkyl-, $C_3$-$C_7$-Ccycloalkyl-, $C_3$-$C_7$-Cycloalkyl-$C_1$-$C_3$-alkylen-, Aryl-$C_1$-$C_6$-alkylen-, Aryl- oder Heteroarylgruppe substituiert ist;

$R_3$ und $R_4$ unabhängig voneinander für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen-, Aryl- oder Heteroarylgruppe stehen;

$R_5$ für eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen-, Aryl- oder Heteroarylgruppe steht;

$R_6$ für ein Wasserstoff- oder Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_3$-$C_7$-Cycloalkoxy-, $C_3$-$C_7$-Cycloalkyl-$C_1$-$C_6$-alkylen-O-,

$C_1$-$C_6$-Fluoralkoxy-, $C_1$-$C_6$-Thioalkyl-, -S(O)-$C_1$-$C_6$-Alkyl-, -S(O)$_2$-$C_1$-$C_6$-Alkyl-, Aryl-, Aryl-$C_1$-$C_6$-alkylen-, Heteroaryl-, Hydroxyl-, Thiol-, Oxo- oder Thiogruppe steht;
$R_7$ für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_3$-$C_7$-Cycloalkoxy-, $C_3$-$C_7$-Cycloalkyl-$C_1$-$C_6$-alkylen-O-, $C_1$-$C_6$-Fluoralkoxy-, Aryl-, Aryl-$C_1$-$C_6$-alkylen- oder Heteroarylgruppe steht;
$R_8$, $R_9$ und $R_{10}$ so definiert sind, daß:
zwei Gruppen $R_8$ gemeinsam eine Bindung oder eine $C_1$-$C_6$-Alkylengruppe bilden können;
zwei Gruppen $R_9$ gemeinsam eine Bindung oder eine $C_1$-$C_6$-Alkylengruppe bilden können;
$R_8$ und $R_9$ gemeinsam eine Bindung oder eine $C_1$-$C_6$-Alkylengruppe bilden können;
$R_8$ und $R_{10}$ gemeinsam eine Bindung oder eine $C_1$-$C_6$-Alkylengruppe bilden können;
$R_9$ und $R_{10}$ gemeinsam eine Bindung oder eine $C_1$-$C_6$-Alkylengruppe bilden können;
$R_{11}$ für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_3$-$C_7$-Cycloalkoxy-, $C_3$-$C_7$-Cycloalkyl-$C_1$-$C_6$-alkylen-O-, $C_1$-$C_6$-Fluoralkoxy-, Hydroxyl-, COOR$_5$-, C(O)NR$_1$R$_2$-, Aryl-$C_1$-$C_6$-alkylen-, Aryl- oder Heteroarylgruppe steht, wobei das Aryl und das Heteroaryl gegebenenfalls durch einen oder mehrere unter einem Halogen und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_3$-$C_7$-Cycloalkoxy-, $C_1$-$C_6$-Fluoralkoxy-, Nitro- oder Cyanogruppe ausgewählte Substituenten substituiert ist;
$R_{12}$ für ein Fluoratom, eine gegebenenfalls durch eine Gruppe $R_{13}$ substituierte $C_1$-$C_6$-Alkylgruppe, eine $C_3$-$C_7$-Cycloalkylgruppe, eine $C_3$-$C_7$-Cycloalkyl-$C_1$-$C_3$-alkylengruppe, eine $C_1$-$C_6$-Fluoralkylgruppe, eine $C_1$-$C_6$-Cycloalk-1,1-diylgruppe, eine gegebenenfalls an einem Stickstoffatom durch eine Gruppe $R_{11}$ substituierte $C_3$-$C_7$-Heterocycloalk-1,1-diylgruppe, eine $C_1$-$C_6$-Alkoxygruppe, eine $C_3$-$C_7$-Cycloalkoxygruppe, eine $C_3$-$C_7$-Cycloalkyl-$C_1$-$C_6$-alkylen-O-Gruppe, eine $C_1$-$C_6$-Fluoralkoxygruppe, eine C(O)NR$_1$R$_2$-Gruppe, eine NR$_1$R$_2$-Gruppe, eine NR$_3$COR$_4$-Gruppe, eine OC(O)NR$_1$R$_2$-Gruppe, eine NR$_3$COOR$_5$-Gruppe, eine NR$_3$CONR$_1$R$_2$-Gruppe, eine Hydroxylgruppe, eine Thiolgruppe, eine Oxogruppe, eine Thiogruppe, eine Aryl-$C_1$-$C_6$-alkylengruppe oder eine Arylgruppe steht, wobei das Aryl gegebenenfalls durch einen oder mehrere unter einem Halogen und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_3$-$C_7$-Cycloalkoxy-, $C_1$-$C_6$-Fluoralkoxy-, Nitro- oder Cyanogruppe ausgewählte Substituenten substituiert ist;
$R_{13}$ für $C_1$-$C_6$-Alkoxy, $C_3$-$C_7$-Cycloalkoxy, $C_3$-$C_7$-Cycloalkyl-$C_1$-$C_6$-alkylen-O-, C(O)NR$_1$R$_2$, NR$_1$R$_2$, NR$_3$COR$_4$, OC(O)NR$_1$R$_2$, NR$_3$COOR$_5$ oder Hydroxyl steht;

wobei das Stickstoffatom bzw. die Stickstoffatome der Verbindung der allgemeinen Formel (I) in oxidierter Form vorliegen kann bzw. können;
wobei das Schwefelatom bzw. die Schwefelatome der Verbindung der allgemeinen Formel (I) in oxidierter Form vorliegen kann bzw. können;
in Basen- oder Säureadditionssalzform sowie in Hydrat- oder Solvatform.

2. Verbindung der Formel (I) nach Anspruch 1, **dadurch gekennzeichnet, daß** $X_1$, $X_2$, $X_3$ und $X_4$ unabhängig voneinander unter einem Wasserstoff- oder Halogenatom oder einer $C_1$-$C_6$-Alkyl-, $C_1$-$C_6$-Fluoralkyl-, $C_1$-$C_6$-Thioalkyl- oder -S(O)$_2$-$C_1$-$C_6$-Alkylgruppe ausgewählt sind;
in Basen- oder Säureadditionssalzform sowie in Hydrat- oder Solvatform.

3. Verbindung der Formel (I) nach Anspruch 1, **dadurch gekennzeichnet, daß**
$X_1$ für ein Wasserstoff- oder Halogenatom oder eine $C_1$-$C_6$-Alkylgruppe steht;
$X_2$ für ein Wasserstoff- oder Halogenatom oder eine $C_1$-$C_6$-Alkyl-, $C_1$-$C_6$-Fluoralkyl- oder -S(O)$_2$-$C_1$-$C_6$-Alkylgruppe steht;
$X_3$ und $X_4$ unabhängig voneinander für ein Wasserstoff- oder Halogenatom oder eine $C_1$-$C_6$-Alkyl-, $C_1$-$C_6$-Fluoralkyl-, $C_1$-$C_6$-Alkoxy-, NR$_1$R$_2$- oder $C_1$-$C_6$-Thioalkylgruppe stehen;
$R_1$ und $R_2$ unabhängig voneinander für eine $C_1$-$C_6$-Alkylgruppe stehen;
in Basen- oder Säureadditionssalzform sowie in Hydrat- oder Solvatform.

4. Verbindung der Formel (I) nach einem der Ansprüche 1 bis 3, **dadurch gekennzeichnet, daß** $Z_1$, $Z_2$, $Z_3$ und $Z_4$ unabhängig voneinander für ein Stickstoffatom oder eine C($R_6$)-Gruppe stehen, wobei mindestens zwei dieser Variablen für eine C($R_6$)-Gruppe stehen; wobei das Stickstoffatom bzw. die Stickstoffatome im Ring, definiert als Stickstoff in der 1-Position, gegebenenfalls durch $R_7$ substituiert ist bzw. sind, wenn das Kohlenstoffatom in 2- oder 4-Position zu diesem Referenzstickstoff durch eine Oxo- oder Thiogruppe substituiert ist; wobei $R_6$ und $R_7$ die in der allgemeinen Formel (I) nach Anspruch 1 angegebene Bedeutung besitzen;
in Basen- oder Säureadditionssalzform sowie in Hydrat- oder Solvatform.

**5.** Verbindung der Formel (I) nach Anspruch 4, **dadurch gekennzeichnet, daß** $Z_1$ und $Z_3$ für eine $C(R_6)$-Gruppe stehen und $Z_2$ und $Z_4$ für ein Stickstoffatom stehen; wobei $R_6$ für ein Wasserstoffatom steht; in Basen- oder Säureadditionssalzform sowie in Hydrat- oder Solvatform.

**6.** Verbindung der Formel (I) nach Anspruch 4, **dadurch gekennzeichnet, daß** $Z_1$, $Z_2$, $Z_3$ und $Z_4$ unabhängig voneinander für ein Stickstoffatom oder eine $C(R_6)$-Gruppe stehen, wobei mindestens eine der Variablen für ein Stickstoffatom steht und die anderen Variablen für eine $C(R_6)$-Gruppe stehen; wobei das Stickstoffatom bzw. die Stickstoffatome im Ring, definiert als Stickstoff in der 1-Position, gegebenenfalls durch $R_7$ substituiert ist bzw. sind, wenn das Kohlenstoffatom in 2- oder 4-Position zu diesem Referenzstickstoff durch eine Oxo- oder Thiogruppe substituiert ist; wobei $R_6$ und $R_7$ die in der allgemeinen Formel (I) nach Anspruch 1 angegebene Bedeutung besitzen;
in Basen- oder Säureadditionssalzform sowie in Hydrat- oder Solvatform.

**7.** Verbindung der Formel (I) nach Anspruch 6, **dadurch gekennzeichnet, daß**
$Z_4$ für ein Stickstoffatom steht und $Z_1$, $Z_2$ und $Z_3$ unabhängig voneinander für eine $C(R_6)$-Gruppe stehen; $R_6$ für ein Wasserstoffatom oder eine $C_1$-$C_6$-Alkyl-, $C_1$-$C_6$-Fluoralkyl- oder $C_1$-$C_6$-Alkoxygruppe steht;
in Basen- oder Säureadditionssalzform sowie in Hydrat- oder Solvatform.

**8.** Verbindung der Formel (I) nach einem der Ansprüche 1 bis 7, **dadurch gekennzeichnet, daß** n gleich 1 ist;
in Basen- oder Säureadditionssalzform sowie in Hydrat- oder Solvatform.

**9.** Verbindung der Formel (I) nach einem der Ansprüche 1 bis 8, **dadurch gekennzeichnet, daß** Y für ein Aryl oder Heteroaryl steht, das gegebenenfalls durch eine oder mehrere unter einem Halogenatom oder einer $C_1$-$C_6$-Alkyl- oder $NR_1R_2$-Gruppe ausgewählte Gruppen substituiert ist;
$R_1$ und $R_2$ gemeinsam mit dem Stickstoffatom, an das sie gebunden sind, eine Azetidinyl-, Pyrrolidinyl-, Piperidinyl-, Azepinyl-, Morpholinyl-, Thiomorpholinyl-, Piperazinyl- oder Homopiperazinylgruppe bilden, wobei diese Gruppe gegebenenfalls durch eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen-, Aryl- oder Heteroarylgruppe substituiert ist;
in Basen- oder Säureadditionssalzform sowie in Hydrat- oder Solvatform.

**10.** Verbindung der Formel (I) nach einem der Ansprüche 1 bis 9, **dadurch gekennzeichnet, daß**
Z für ein über das Stickstoffatom gebundenes cyclisches Amin der Formel:

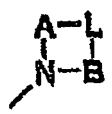

steht, worin A für eine gegebenenfalls durch eine oder zwei Gruppen $R_8$ substituierte $C_1$-$C_4$-Alkylengruppe steht;
B für eine gegebenenfalls durch eine oder zwei Gruppen $R_9$ substituierte $C_1$-$C_4$-Alkylengruppe steht;
L für eine Bindung oder ein Sauerstoffatom steht;

wobei das Stickstoffatom des cyclischen Amins Z in N-Oxid-Form vorliegen kann;
wobei die Kohlenstoffatome des cyclischen Amins Z gegebenenfalls durch eine Gruppe $R_{12}$ substituiert sind;
$R_8$ und $R_9$ so definiert sind, daß:

zwei Gruppen $R_8$ gemeinsam eine Bindung bilden können oder
zwei Gruppen $R_9$ gemeinsam eine Bindung bilden können oder
$R_8$ und $R_9$ gemeinsam eine Bindung bilden können;

$R_{12}$ für eine $NR_1R_2$-, $NR_3COOR_5$- oder Hydroxylgruppe steht;
$R_1$ und $R_2$ unabhängig voneinander für ein Wasserstoffatom stehen;
$R_3$ für ein Wasserstoffatom steht;
$R_5$ für eine $C_1$-$C_6$-Alkylgruppe steht;
in Basen- oder Säureadditionssalzform sowie in Hydrat- oder Solvatform.

**11.** Verbindung der Formel (I) nach Anspruch 10,
**dadurch gekennzeichnet, daß**
Z für ein unter Azetidin, Pyrrolidin, Piperidin, Morpholin, Azabicylo[3.1.0]hexan und Azabicylo-[3.2.0]heptan ausgewähltes cyclisches Amin steht;
wobei das Stickstoffatom des cyclischen Amins Z in N-Oxid-Form vorliegen kann;
wobei die Kohlenstoffatome des cyclischen Amins Z gegebenenfalls durch eine Gruppe $R_{12}$ substituiert sind;
$R_{12}$ für eine $NR_1R_2$-, $NR_3COOR_5$- oder Hydroxylgruppe steht;
$R_1$ und $R_2$ unabhängig voneinander für ein Wasserstoffatom stehen;
$R_3$ für ein Wasserstoffatom steht;
$R_5$ für eine $C_1$-$C_6$-Alkylgruppe steht;
in Basen- oder Säureadditionssalzform sowie in Hydrat- oder Solvatform.

**12.** Verbindung der Formel (I) nach Anspruch 11, **dadurch gekennzeichnet, daß**
Z für ein unter Azetidin, Pyrrolidin, Piperidin, Morpholin, Azabicylo[3.1.0]hexan und Azabicylo-[3.2.0]heptan ausgewähltes cyclisches Amin steht;
wobei die Kohlenstoffatome des Azetidins gegebenenfalls durch eine Hydroxylgruppe substituiert sind;
wobei die Kohlenstoffatome des Pyrrolidins gegebenenfalls durch eine $NR_1R_2$-, $NR_3COOR_5$- oder Hydroxylgruppe substituiert sind; wobei das Stickstoffatom des Pyrrolidins in N-Oxid-Form vorliegen kann;
$R_1$ und $R_2$ unabhängig voneinander für ein Wasserstoffatom stehen;
$R_3$ für ein Wasserstoffatom steht;
$R_5$ für eine $C_1$-$C_6$-Alkylgruppe steht;
in Basen- oder Säureadditionssalzform sowie in Hydrat- oder Solvatform.

**13.** Verfahren zur Herstellung einer Verbindung der Formel (I) nach einem der Ansprüche 1 bis 12,
**dadurch gekennzeichnet, daß** man eine Verbindung der allgemeinen Formel (IV)

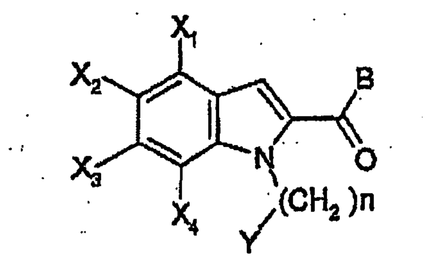
(IV)

worin $X_1$, $X_2$, $X_3$, $X_4$, Y und n die in der allgemeinen Formel (I) nach Anspruch 1 angegebene Bedeutung besitzen und B für ein Chloratom steht,
in einem Lösungsmittel mit einem Amin der allgemeinen Formel (V)

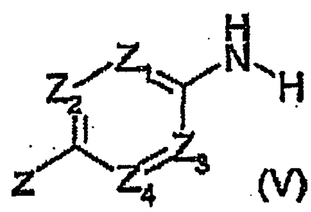
(V)

worin $Z_1$, $Z_2$, $Z_3$, $Z_4$ und Z die in der allgemeinen Formel (I) nach Anspruch 1 angegebene Bedeutung besitzen, umsetzt.

**14.** Verfahren zur Herstellung einer Verbindung der Formel (I) nach einem der Ansprüche 1 bis 12, **dadurch gekennzeichnet, daß** man eine Verbindung der allgemeinen Formel (IV)

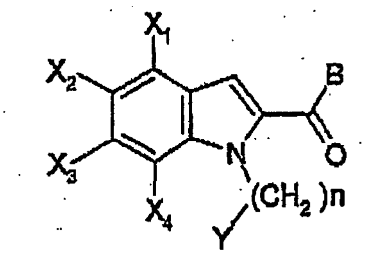

(IV)

worin $X_1$, $X_2$, $X_3$, $X_4$, Y und n die in der allgemeinen Formel (I) nach Anspruch 1 angegebene Bedeutung besitzen und B für eine Hydroxylgruppe steht, in Gegenwart eines Kupplungsmittels und einer Base in einem Lösungsmittel mit einem Amin der allgemeinen Formel (V)

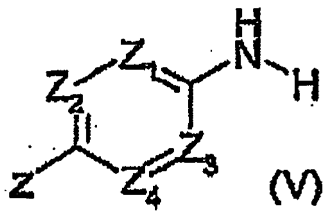

(V)

worin $Z_1$, $Z_2$, $Z_3$, $Z_4$ und Z die in der allgemeinen Formel (I) nach Anspruch 1 angegebene Bedeutung besitzen, umsetzt.

**15.** Verfahren zur Herstellung einer Verbindung der Formel (I) nach einem der Ansprüche 1 bis 12, **dadurch gekennzeichnet, daß** man eine Verbindung der allgemeinen Formel (VI)

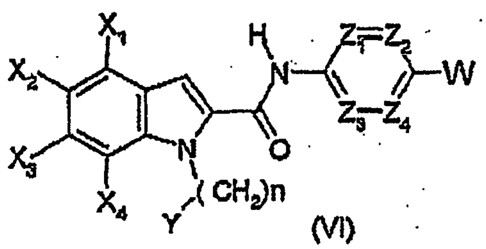

(VI)

worin $X_1$, $X_2$, $X_3$, $X_4$, $Z_1$, $Z_2$, $Z_3$, $Z_4$, Y und n die in der allgemeinen Formel (I) nach Anspruch 1 angegebene Bedeutung besitzen und W für ein Halogenatom steht, in Gegenwart eines Amins ZH, worin Z für ein cyclisches Amin gemäß der in der allgemeinen Formel (I) nach Anspruch 1 angegebenen Definition steht, umsetzt.

**16.** Amin der allgemeinen Formel (V), ausgewählt unter den folgenden Aminen:

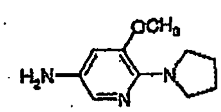
(Va)

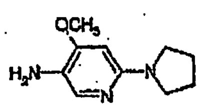
(Vb)

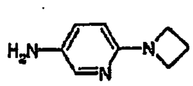
(Vc)

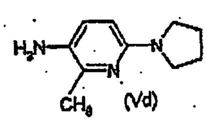
(Vd)

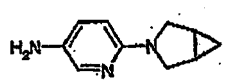
(Vf)

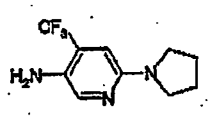
(Vg)

in Basen- oder Säureadditionssalzform.

**17.** Arzneimittel, **dadurch gekennzeichnet, daß** es eine Verbindung der Formel (I) nach einem der Ansprüche 1 bis 12 oder ein pharmazeutisch annehmbares Salz oder auch ein Hydrat oder ein Solvat der Verbindung der Formel (I) enthält.

**18.** Pharmazeutische Zusammensetzung, **dadurch gekennzeichnet, daß** sie eine Verbindung der Formel (I) nach einem der Ansprüche 1 bis 12 oder ein pharmazeutisch annehmbares Salz oder auch ein Hydrat oder ein Solvat dieser Verbindung sowie mindestens einen pharmazeutisch annehmbaren Hilfsstoff enthält.

**19.** Verwendung einer Verbindung der Formel (I) nach einem der Ansprüche 1 bis 12 zur Herstellung eines Arzneimittels zur Prävention oder Behandlung von Pathologien, an denen die Rezeptoren vom TRPV1-Typ beteiligt sind.

**20.** Verwendung einer Zusammensetzung der Formel (I) nach einem der Ansprüche 1 bis 12 zur Herstellung eines Arzneimittels zur Prävention oder Behandlung von Schmerzen, Entzündungen, urologischen Störungen, gynäkologischen Störungen, gastrointestinalen Störungen, respiratorischen Störungen, Psoriasis, Pruritis, Haut-, Augen- oder Schleimhautreizungen, Herpes oder Zona oder zur Behandlung von Depression oder Diabetes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006072736 A
- JP 2001151771 A
- WO 07010144 A
- WO 05028452 A
- WO 02048152 A
- WO 06040522 A
- WO 04052869 A
- JP 7051121 A
- WO 05035526 A
- WO 07011284 A
- WO 04062665 A
- GB 870027 A
- US 4104385 A
- WO 2006024776 A
- JP 2004175739 B
- WO 0248152 A
- WO 03080610 A
- US 20060281772 A
- WO 2007010144 A


### Littérature non-brevet citée dans la description


- **J. March.** Advances in Organic Chemistry. Wiley Interscience, 2001
- **D. Knittel.** *Synthesis,* 1985, vol. 2, 186
- **T.M. Williams.** *J.Med.Chem.,* 1993, vol. 36 (9), 1291
- **Kolasa T.** *Bioorg.Med.Chem.,* 1997, vol. 5 (3), 507
- **Abramovitch R.** *Synth. Commun.,* 1995, vol. 25 (1), 1
- **O. Mitsonobu.** *Synthesis,* 1981, 1-28
- **Murakami Y.** *Chem.Pharm.Bull.,* 1995, vol. 43 (8), 1281
- **S.L. Buchwald.** *J.Am.Chem.Soc.,* 2002, vol. 124, 11684
- **J.F. Hartwig.** *Angew Chem. Int. Ed.,* 2005, vol. 44, 1371-1375
- *Pharmazie,* 1990, vol. 45, 346
- **Carling R.W. et al.** *J.Med.Chem.,* 2004, 1807-1822
- **Russel M.G.N. et al.** *J. Med. Chem.,* 2005, 1367-1383
- *Heterocycles,* 1977, vol. 6 (12), 1999-2004
- *J.Chem.Soc. Perkin trans 1,* 1973, 68-69
- *Bioorg.&Med.Chem. Lett.,* 2005, vol. 15 (8), 2093
- *J.Med.Chem,* 1967, vol. 10 (4), 621